(19) Europäisches Patentamt
European Patent Office
Office européen des brevets

(11) Veröffentlichungsnummer: **0 101 027**
**A1**

# (12) EUROPÄISCHE PATENTANMELDUNG

(21) Anmeldenummer: 83107729.2

(22) Anmeldetag: 05.08.83

(51) Int. Cl.³: A 61 K 9/22
A 61 K 9/18

(30) Priorität: 18.08.82 DE 3230736

(43) Veröffentlichungstag der Anmeldung:
22.02.84 Patentblatt 84/8

(84) Benannte Vertragsstaaten:
BE CH DE FR GB IT LI NL SE

(71) Anmelder: Rupprecht, Herbert, Prof. Dr.
Machthildstrasse 47
D-8400 Regensburg(DE)

(72) Erfinder: Rupprecht, Herbert, Prof. Dr.
Machthildstrasse 47
D-8400 Regensburg(DE)

(74) Vertreter: Kraus, Walter, Dr. et al,
Patentanwälte Dres. Kraus & Weisert Irmgardstrasse 15
D-8000 München 71(DE)

## (54) An Siliciumdioxid als Träger gebundene Wirkstoffe, Verfahren zu ihrer Herstellung und ihre Verwendung.

(57) Die Erfindung betrifft an Siliciumdioxid als Träger gebundene Wirkstoffe, die entweder fest verankert sind obder hydrolytisch abspaltbar sind. Der wirksame Bestandteil kann zum Beispiel der Rest eines Desinfektionsmittels, einer geruchsbindenden Substanz oder eines pharmazeutischen Wirkstoffs sein. Wenn die Wirkstoffe hydrolytisch abspaltbar sind, wird beim Kontakt mit wasserhaltiger Flüssigkeit oder mit Luftfeuchtigkeit der Wirkstoff allmählich freigesetzt und kann seine Wirkung entfalten. Ein Verfahren zur Herstellung der an Siliciumdioxid als Träger gebundenen Wirkstoffe wird ebenfalls beschrieben.

EP 0 101 027 A1

0101027

Die Erfindung betrifft an Siliciumdioxid als Träger gebundene Wirkstoffe, Verfahren zu ihrer Herstellung und ihre Verwendung.

Die vorliegende Erfindung betrifft trägergebundene Wirkstoffe sowie Verfahren zu ihrer Herstellung.

Anorganische Trägermaterialien vom Typ der amorphen Kieselsäuren, des amorphen Titandioxids und Aluminiumoxids erfreuen sich wegen ihrer günstigen technologischen Eigenschaften, wie z. B. große spezifische Oberfläche, überschaubare Wechselwirkungen, gute Verarbeitbarkeit, im Bereich der pharmazeutischen Technologie einer breiten Verwendung. (Remington's Pharmaceutical Sciences 15.Ed., Mack Publishing Comp., Easton, Pa 1975).

In jüngster Zeit werden sie zunehmend zur Steuerung der Wirkstoffliberation, z.B. für peroral verabreichte Retardarzneimittel (Rupprecht, H., Unger, K., Kramer, H., Kircher W., "Microencapsulation", New Tech. Appl., Proc. Int. 3rd (1979)), oder als Aufziehsubstanzen für sehr niedrig dosierte Wirkstoffe (H. Rupprecht in "Topics in Pharmaceutical Sciences", D.D. Breimer, P. Speiser Ed., Elsevier, Amsterdam, New York, Oxford 1981 p. 443 ff) oder zur Beschleunigung des Auflösevorgangs schwer löslicher Wirkstoffe eingesetzt. Die Arzneimittel behalten dabei nach mechanischer Abscheidung, physikalischer Adsorption oder Bindung über Ionenaustausch an den Träger ihre chemische Identität bei. Eine chemische Verankerung von Wirksubstanzen mit dem Ziel der Arzneisteuerung ist dagegen bisher auf anorganischen Trägern vom Typ des kolloiden $SiO_2$, $TiO_2$ oder $Al_2O_3$ nicht vorgenommen worden.

Das Prinzip trägergebundener Wirkstoffe hat im Bereich der Chromatographie und der Enzymtechnologie zunehmend an Bedeutung gewonnen, und, wie die Erfahrungen mit trägergebundenen organofunktionellen Gruppen bei chromatographischen Trennverfahren und in der Enzymtechnologie oder zur Verbesserung der Partikelhaftung zeigen (Salmona, M., Saronio, C., Garattini, S., "Insolubilized Enzymes", Weco York 1974; Unger, K., "Porous Silica", Amsterdam, Oxford, New York 1979;), weist ein solches Prinzip viele Vorteile auf: Neben einer besseren chemischen Stabilität und erhöhter Trennleistung sowohl bei Enzymen als auch in der Chromatographie kommen solche trägergebundenen Substanzen Produktionsprozessen technisch besser entgegen. So übertrifft z.B. die Standzeit trägergebundener Enzyme häufig bei weitem jene, die Enzyme in flüssigen Reaktoren zeigen.

In jüngster Zeit finden sich in der Literatur auch Hinweise darauf, daß pharmazeutische Wirksubstanzen chemisch fest verankert gegenüber biologischen Modellen eine hohe Aktivität aufweisen (Venter, J.C., Dissertation Univ. Calif., San Diego 1975; Wolfish, J.H., Janauer, G.E., Water, Air, Soil Pollut. 12, 477 (1979)): So lösen an Glasoberflächen verankerte Sympathikomimetika an isolierten Geweben ähnliche physiologische Effekte aus wie die entsprechenden gelösten Arzneimittel.

Auf der Basis organischer Resinate wurden antimikrobiell wirksame Prinzipien entwickelt, bei denen quartäre Ammoniumverbindungen, chemisch fest verankert, Mikroorganismen abzutöten vermögen. (Walfish J., Janauer G., Water, Air, Soil Pollut. 12, 477 (1979)).

Über die Anwendung poröser oder kolloider anorganischer Substanzen - insbesondere vom Typ der amorphen Kieselsäuren - als Träger zur chemischen Verankerung von pharmazeutisch relevanten Wirksubstanzen liegen bisher noch keine Hinweise vor. Allerdings findet sich eine große Zahl von Angaben über Chemisorption verschiedener organischer

funktioneller Gruppen an Kieselsäureoberflächen, insbesondere für chromatographische Zwecke (Unger, K., "Porous Silica", Amsterdam, Oxford, New York 1979; Brust, O.E., Halasz, J., J. Chromatogr. 83, 15 (1973)).

Der vorliegenden Erfindung liegt die Aufgabe zugrunde, therapeutisch wirksame, z.B. antimikrobiell oder lokalanästhetisch wirksame oder diagnostisch einsetzbare, trägergebundene Wirkstoffe sowie ein Verfahren zur Herstellung solcher trägergebundener Wirkstoffe zur Verfügung zu stellen. Von trägergebundenen Wirkstoffen sind eine Reihe von Vorteilen gegenüber vergleichbaren löslichen Substanzen zu erwarten, die sowohl ihre Stabilität als auch ihre Wirkung betreffen.

Neben bequemer technologischer Handhabung, z.B. Verpreßbarkeit zu Tabletten, Einarbeitung in Suspensionen, Kapseln, Sprühprodukte, entfalten fest verankerte Gruppen nur lokal eine Wirkung. Durch die neuen erfindungsgemäßen unlöslichen Verbindungen soll die systematische Belastung des Organismus dadurch reduziert werden, daß sie nur lokal wirksam werden. Dies bedeutet, daß sie nur in unmittelbarem Kontakt mir Rezeptoren am Körper oder Mikroorganismen ihre Wirkung entfalten, nicht aber durch Abspaltung vom Träger oder durch die Gesamtresorption von Träger und Wirkstoff in den Körper gelangen.

Es ist bekannt, daß alle Konservierungsmittel und Lokalanästhetika eine nicht unbeträchtliche Toxizität besitzen, wodurch ihre Einsatzmöglichkeit gegebenenfalls erheblich beschränkt wird. Die kolloide Teilchengröße der von uns verwendeten Trägermaterialien ermöglicht eine optimale Verteilung und Stabilität in flüssigen Dispersionen sowie einen entsprechend guten, gleichmäßigen Kontakt mit den Grenzflächen der Mikroorganismen (z.B. Bakterienzellwand).

Aufgabe der vorliegenden Erfindung ist es somit, pharmakologisch wirksame Substanzen an amorphen anorganischen Trägern chemisch zu verankern. Die entsprechenden Chemiesorbate sollen bei der Anwendung entweder durch kontrollierte Abspaltung des Wirkstoffs ("pro-drug-Prinzip") oder lokal durch Kontakt mit dem Gewebe bzw. mit Körperflüssigkeiten bei fester Verankerung der aktiven Gruppen an den Trägern ihre Wirkung entfalten. Hierbei wird angestrebt, die hergestellten trägergebundenen pharmazeutischen Wirkstoffe physikalisch-chemisch und biologisch eindeutig zu charakterisieren und zu normieren.

Erfindungsgemäß soll somit eine chemische Verankerung von Wirkstoffen, insbesondere von pharmazeutisch wirksamen Stoffen, zur Verfügung gestellt werden.

Die erfindungsgemäße Verankerung vor Wirkstoffen an festen $SiO_2$-Trägern kann entweder so erfolgen, daß stabile Si-C-Brücken als Anker zum Trägermaterial dienen, oder so, daß zwischen Trägeroberfläche und dem Wirkstoffmolekül eine spaltbare Si-O-C-Gruppe oder Si-N-C-Gruppe oder Si-S-C-Gruppe vorliegt. Die entsprechenden Chemisorbate sollen bei der Anwendung entweder in Kontakt mit Geweben bzw. mit Körperflüssigkeiten bei fester Verankerung der aktiven Gruppe wirksam werden oder bei den instabilen letztgenannten Gruppen eine kontrollierbare Abspaltung der Wirkstoffe ermöglichen.

Die notwendigen erfindungsgemäßen Chemisorptionsreaktionen gehen im Prinzip von zwei Wegen aus:

a) Feste Verankerung: In einem ersten Schritt wird an die $SiO_2$-Oberfläche ein Chlorsilan (oder Ethoxysilan) mit geeigneter funktioneller Gruppe (=Spacer) eingeführt, wie es im folgenden schematisch dargestellt ist:

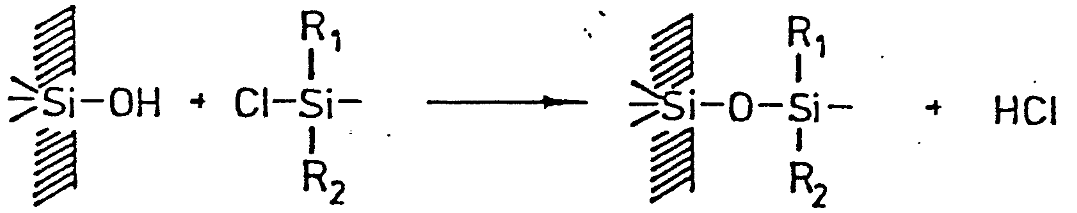

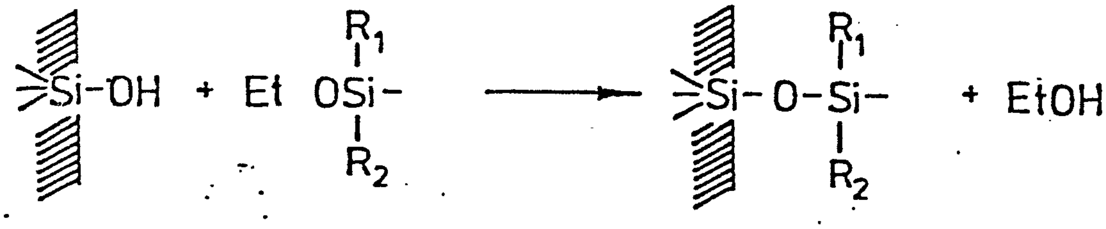

An reaktionsfähigen Gruppen dieser primär verankerten "Spacer" können weitere chemische Reaktionsschritte vollzogen werden.

b) Für die Knüpfung spaltbarer Si-O-C-, Si-O-S- und Si-N-C-Bindungen wird in einem ersten Reaktionsschritt die Oberfläche der Kieselsäure aktiviert.

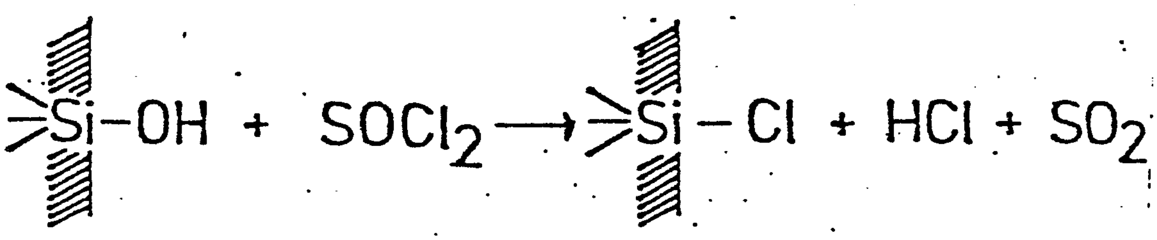

An der reaktiven Oberfläche ist dann die Umsetzung von Substanzen mit geeigneten funktionellen Gruppen möglich.

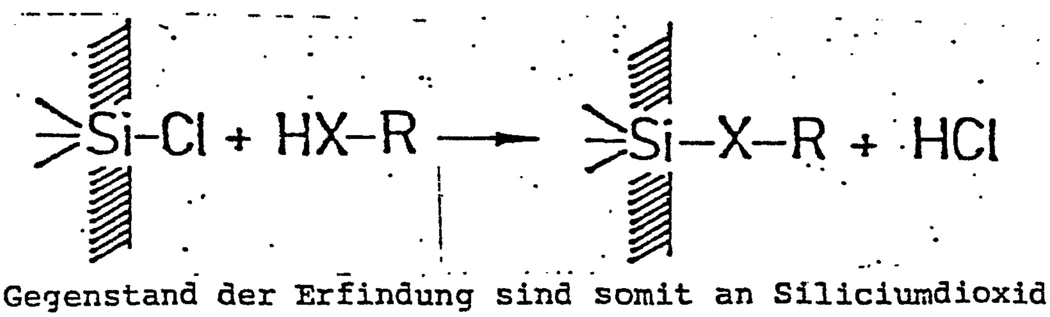

Gegenstand der Erfindung sind somit an Siliciumdioxid als Träger gebundene pharmazeutische Wirkstoffe der folgenden Formeln I, II oder III:

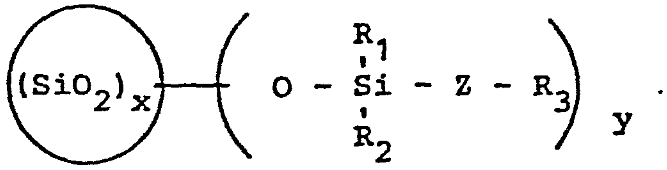 (I)

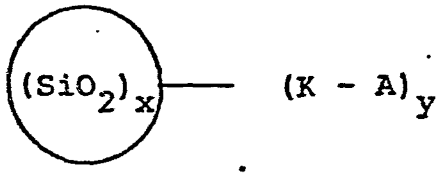 (II)

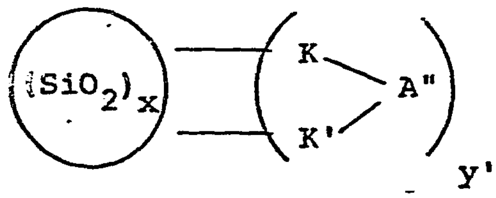 (III)

worin 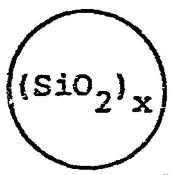 hochdisperse Teilchen bedeutet, y und y' die pro Menge oder Oberflächeneinheit des Trägers verankerten Gruppen in Mol bedeuten (mMol/g oder mMol/m²), y im Bereich von 0,1 bis 1 und y' im Bereich von 0,1 bis 0,5 liegen, Z $-(CH_2)_n-$, worin n für 0 bis 18 steht, oder einen Rest der Formeln

$$-[(CH_2)_3-O]_{n'}, \quad -CH_2-\underset{OH}{CH}-CH_2-$$

oder

$$-HN-(CH_2)_3-NH-$$

bedeutet, worin n' für 1 bis 3 steht,

$R_1$ und $R_2$ gleich oder verschieden sein können und eine Methyl- oder eine Hydroxylgruppe bedeuten,

$R_3$ den einwertigen Rest eines Wirkstoffs bedeutet, mit der Maßgabe, daß, wenn Z eine Alkylengruppe bedeutet, in der n für 0 steht, der Wirkstoff unmittelbar mit einem seiner Kohlenstoffatome mit dem Siliciumatom verknüpft ist,

K und K' O, S oder >N-A' bedeuten, wobei in der Formel III die beiden Substituenten K und K' gleich oder unterschiedlich sein können,

A den einwertigen Rest eines Wirkstoffs der Formeln IV, V oder VI

H - S-A (IV)

H - O-A (V)

H - N-A (VI)
      |
      A'

bedeutet,

wobei A' ein Wasserstoffatom bedeutet oder wobei in der Formel VI A und A' zusammen mit dem Stickstoffatom, an das sie gebunden sind, den Rest eines Wirkstoffmoleküls bedeuten,

A" den zweiwertigen Rest eines Wirkstoffs der Formel VII

H - K\
        A" (VII)
H - K'/

bedeutet,

und wobei bei der Verankerung mit dem Träger bei den Wirkstoffen der Formel IV oder VI ein Wasserstoffatom durch den Träger ersetzt wurde und bei dem Wirkstoff der Formel VII zwei Wasserstoffatome durch den Träger ersetzt wurden.

Die Erfindung betrifft weiterhin ein Verfahren zur Herstellung der erfindungsgemäßen Verbindungen I, II oder III, das dadurch gekennzeichnet ist, daß man

1) zur Herstellung von Verbindungen der Formel I

a) eine Verbindung der Formel XVII

$$\begin{array}{c} R_1 \\ | \\ R_4 - Si - Z - R_3 \\ | \\ R_2 \end{array} \qquad \text{(XVII)}$$

worin $R_1$, $R_2$ und $R_3$ die oben gegebenen Bedeutungen besitzen und $R_4$ eine $C_1$-$C_3$-Alkoxygruppe oder ein Chloratom bedeutet, mit der Oberfläche des Siliciumdioxids in an sich bekannter Weise kondensiert oder

b) einen reaktiven Spacer der folgenden Formel XVIII

$$\begin{array}{c} R_4 \\ | \\ R_4 - Si - Z - \text{funktionelle Gruppe} \\ | \\ R_4 \end{array} \qquad \text{(XVIII)}$$

in der $R_4$ die oben gegebene Bedeutung besitzt, an der Oberfläche der $SiO_2$-Teilchen durch Kondensation in an sich bekannter Weise verankert und danach den Wirkstoff am Spacer in einem oder mehreren Reaktionsschritten in an sich bekannter Weise einführt oder

c) eine Vorstufe des Wirkstoffs, gegebenenfalls über einen geeigneten Spacer, mit der Oberfläche des $SiO_2$ verknüpft und in einem weiteren Reaktionsschritt in die Wirkform umsetzt

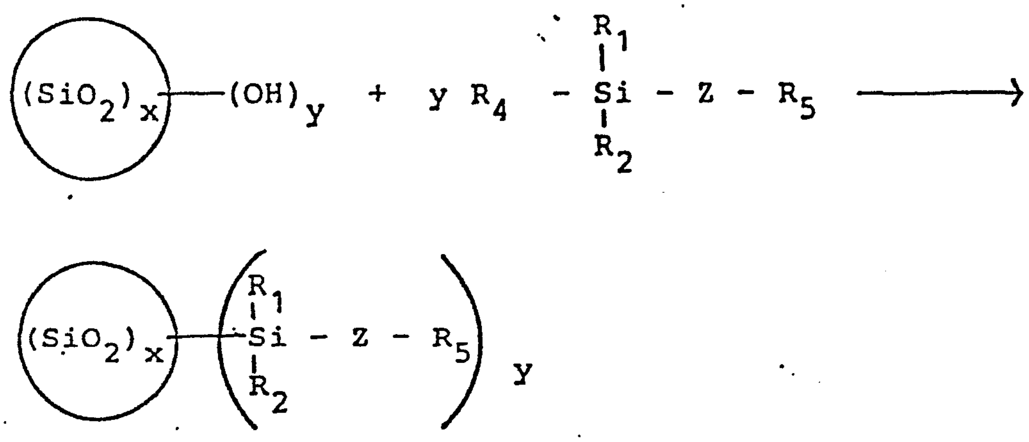

worin $R_1$, $R_2$ und $R_4$ die oben gegebene Bedeutung besitzen und $R_5$ die Vorstufe eines Wirkstoffrestes bedeutet

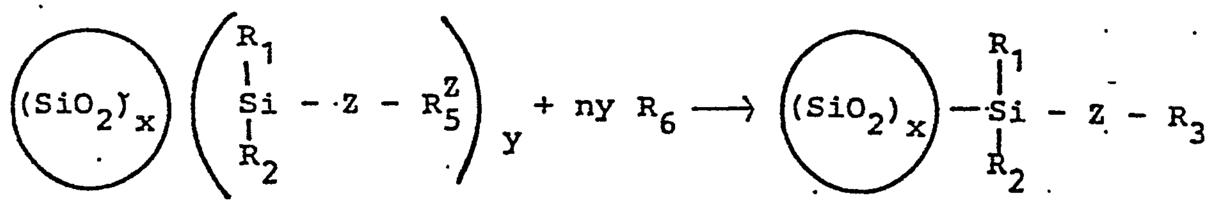

worin $R_6$ ein Reagenz zur Umwandlung der Wirkstoff-Vorstufe $R_5$ in die Wirkform $R_3$ bedeutet und $R_5$ mit η-Molekülen $R_6$, entsprechend der Wertigkeit von $R_5$ reagieren kann.

2) zur Herstellung von Verbindungen der Formel II wasserfreie Kieselsäure in an sich bekannter Weise in Benzol mit Thionylchlorid gemäß folgender Gleichung 1 umsetzt

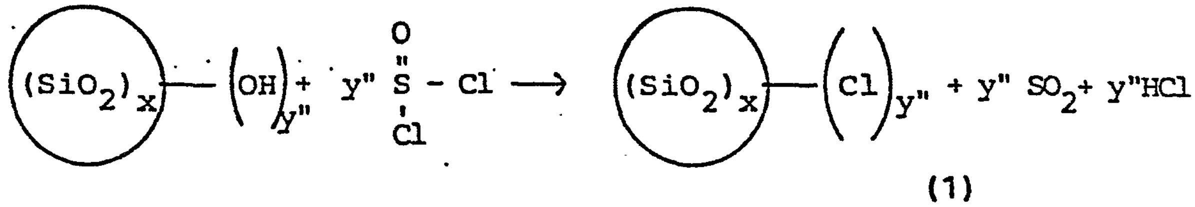

(1)

wobei y" die pro g oder m² an der $SiO_2$-Oberfläche umgesetzte Menge bedeutet, und nach Entfernung der flüchtigen Reaktionsprodukte $SO_2$ und HCl mit einer Verbindung der Formel H - K - A gemäß folgender Gleichung 2

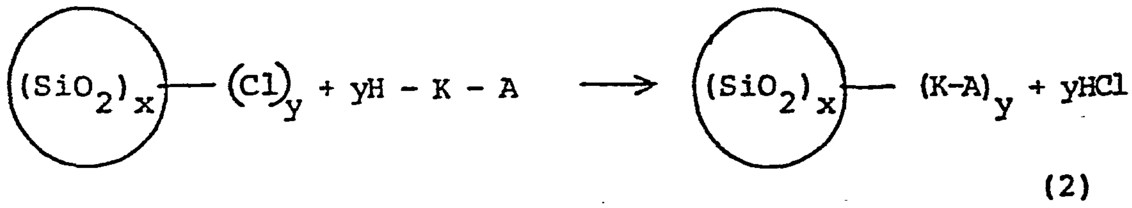

(2)

umsetzt, wobei y ≤ y", A und x die oben gegebenen Bedeutungen besitzen, und das Reaktionsprodukt in an sich bekannter Weise isoliert,

3) zur Herstellung von Verbindungen der Formel III Kieselsäure in an sich bekannter Weise in Benzol mit Thionylchlorid gemäß Gleichung 1 umsetzt und das erhaltene Reaktionsprodukt mit einer Verbindung der Formel gemäß folgender Gleichung 3

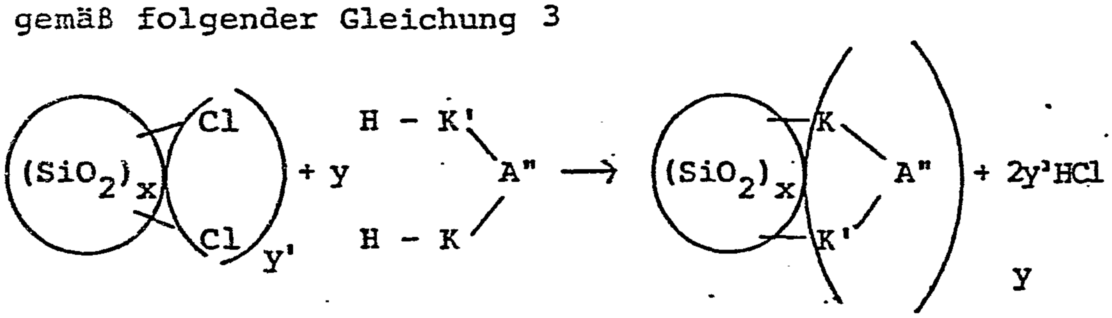

(3)

umsetzt, wobei $y' \leq \frac{y''}{2}$ bedeutet.

Die Erfindung betrifft weiterhin die Verwendung der Verbindungen nach Anspruch 1 als Desinfektionsmittel, geruchsbindende Substanz, spezifische Antidote für den peroralen Gebrauch, Lokalanästhetika, Lichtschutzmittel, als Arzneimittel mit retardierter Wirkstoffreisetzung, als Mittel, bei dem der unangenehme Geschmack oder Geruch des Wirkstoffs beseitigt oder abgemildert ist, und als stabilisierte Arzneiform für Wirkstoffe mit hohem Dampfdruck (flüchtige Substanzen).

Überraschenderweise hat sich gezeigt, daß es möglich ist, Wirkstoffe an $SiO_2$-Oberflächen entweder fest zu verankern oder so zu verankern, daß die wirksamen Gruppen intakt, gegebenenfalls protrahiert abgespalten werden und daß die pharmakologische Wirksamkeit erhalten bleibt. Im folgenden ist eine Übersicht über Wirkstoffe gegeben, die erfindungsgemäß an Siliciumdioxidträger gebunden werden können:

BAD ORIGINAL

I. Hydrolysestabil verankerte Wirkstoffe

In den an $SiO_2$ als Träger gebundenen Wirkstoffen der Formel I ist das gesamte Molekül der Formel I wirksam. Der wirksame Bestandteil, d.h. $R_3$, kann den Rest eines Desinfektionsmittels, einer geruchsbindenden Substanz, eines pharmazeutischen Wirkstoffs, beispielsweise eines Antidots für den peroralen Gebrauch, zum Beispiel für Schwermetallbindungen, oder eines Lokalanästhetikums bedeuten.

Beispiele für Desinfektionsmittel sind solche für die Haut, die Schleimhaut, den gastrointestinalen Trakt, wie z.B. quaternäre Ammoniumverbindungen, Phenole, Hydroxybenzoesäureester und Organo-Quecksilberverbindungen.

Beispiele für geruchsbindende Substanzen sind quaternäre Ammoniumverbindungen. Beispiele für spezifische Antidote für den peroralen Gebrauch sind Cystein, EDTA. Beispiele für Lokalanästhetika sind Polydocanol (Thesit ®), Benzocain oder Lidocain.

II. Hydrolytisch abspaltbare Gruppe ("pro-drug-Prinzip")

Die an $SiO_2$ als Träger gebundenen Wirkstoffe der Formeln II oder III werden bei Kontakt mit wasserhaltiger Flüssigkeit oder mit Luftfeuchtigkeit allmählich freigesetzt und entfalten dann als solche ihre Wirkung.
Die Wirkstoffe können dabei kühlende, desinfizierende, geruchsbindende oder geruchsüberdeckende Wirkung haben. Man kann das Prinzip auch anwenden, um eine retardierte Wirkstoffreisetzung bei peroralen und rektalen Arzneiformen zu erreichen, beispielsweise mit Codein. Dieses Prinzip ist auch anwendbar, um unangenehmen Geruch oder Geschmack von Wirkstoffen zu beseitigen oder abzumildern, wie zum Beispiel von Chinin, p-Chlorcresol, oder zur Stabilisierung flüchtiger Substanzen in festen Arzneiformen, wie zum Beispiel Menthol und Thymol.

Die hydrolysestabil verankerten Wirkstoffe besitzen den Vorteil, daß sie vom Körper nicht resorbiert werden und dadurch eine systemische Belastung unterbleibt. Sie wirken nur an den Stellen im Körper, wo sie tatsächlich appliziert werden. Die chemisorbierten Wirkstoffe werden, nachdem sie ihre Wirkung entfaltet haben, wieder ausgeschieden (aus dem Gastro-Intestinal-Trakt) oder bei der Reinigung der Haut entfernt. eine Kumulation im Körper ist nicht möglich, da sie am Träger fest verankert sind.

Die erfindungsgemäßen Verbindungen sind definierte Verbindungen, und die Struktur der einzelnen Gruppen ist exakt erfaßbar und normierbar, zum Beispiel durch Elementaranalyse, IR-Spektroskopie, UV-Spektroskopie, Austauschkapazität, mikrobiologische Wirksamkeit und Identifizierung in NMR nach Abbau der Matrix.

In den erfindungsgemäßen Verbindungen der Formeln I, II und III wurde das Siliciumdioxid schematisch dargestellt. Erfindungsgemäß verwendet man hochdisperse, vorzugsweise durch Flammenpyrolyse hergestellte Teilchen, die einen $SiO_2$-Gehalt von über 99,8% aufweisen. Sie besitzen eine primäre Teilchengröße von 8 bis 30 nm, vorzugsweise 10 bis 20 nm, und eine BET-Oberfläche von 50 bis 500 m², vorzugsweise 100 bis 400m², besonders bevorzugt 200 bis 250 m². Die Teilchen besitzen auf ihrer Oberfläche ⫶ SiOH-gruppen, die über relativ schwache Wasserstoffbrücken miteinander verknüpft sind.

In den Verbindungen der Formeln I, II und III bedeuten y und y' die pro Menge des Trägers verankerten Gruppen in Mol, wobei die Angabe, je nachdem, in mMol/g oder mMol/m² erfolgt. y und y' sind die maximalen Belegungswerte und werden durch den Platzbedarf und die funktionellen Ankergruppen bestimmt. y liegt im Bereich von 0,1 bis 1, vorzugsweise im Bereich von 0,2 bis 0,6 und besonders bevorzugt im Bereich von 0,3 bis 0,5, mMol/g. y' liegt im Bereich von 0,1 bis 0,5, bevorzugt im Bereich von 0,1 bis 0,3 mMol/g.

Z bedeutet in den Verbindungen der Formel I eine geradlinige Alkylengruppe der Formel $-(CH_2)_n-$, worin n für 0 bis 18, vorzugsweise für 1 bis 10 und besonders bevorzugt für 1 bis 5, am meisten bevorzugt für 1, 2 oder 3, steht. Die Zahl n kann auch für 0 stehen. In diesem Fall ist der Substituent $R_3$ über eines seiner Kohlenstoffatome direkt an das Siliciumatom gebunden. Z kann weiterhin einen Rest der Formeln

$$-[(CH_2)_3-O]_{n'},\quad -CH_2-\underset{\displaystyle OH}{CH}-CH_2-$$

oder $$-HN-(CH_2)_3-NH-$$

bedeuten, worin n' für 1 bis 3, vorzugsweise für 1, steht.

In der Formel I können die Substituenten $R_1$ und $R_2$ eine Methylgruppe oder eine Hydroxylgruppe sein. Besonders bevorzugte Verbindungen sind solche, worin $R_1$ und $R_2$ beide eine Hydroxylgruppe bedeuten oder worin $R_1$ eine Methylgruppe und $R_2$ eine Hydroxylgruppe bedeuten.

$R_3$ bedeutet den einwertigen Rest eines hydrolysestabil verankerten Wirkstoffs, vorzugsweise eines pharmazeutischen Wirkstoffs, wobei der Wirkstoff über ein Kohlenstoffatom, ein Sauerstoffatom, ein Stickstoffatom oder ein Schwefelatom an Z gebunden sein kann. Wenn in der Verbindung der Formel I Z nicht verhanden ist, muß der Rest des Wirkstoffs, $R_3$, unmittelbar mit einem seiner Kohlenstoffatome mit dem Siliciumatom verknüpft sein. Beispiele für $R_3$ sind:

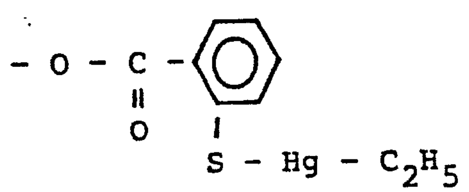

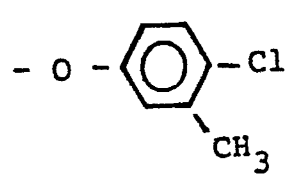

$$-O\,(C_2H_4O)_{9-12}-C_{12}H_{25}$$

In den Verbindungen der Formeln II oder III bedeuten K und K' ein Sauerstoffatom oder ein Schwefelatom oder die Gruppierung N-A'. A bedeutet den einwertigen Rest eines Wirkstoffs der Formeln IV, V oder VI:

H - S-A, N - O-A oder H - N-A
A'

(IV) (V) (VI)

wobei A' ein Wasserstoffatom bedeutet oder wobei in der Formel VI A und A' zusammen mit dem Stickstoffatom, an das sie gebunden sind, den Rest eines Wirkstoffmoleküls bedeuten. In den Verbindungen der Formel III bedeutet A" den zweiwertigen Rest eines Wirkstoffs der Formel VII

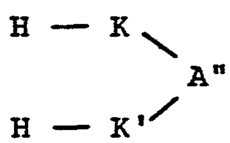

(VII)

Ein Beispiel für einen Wirkstoff der Formel IV ist Rhodanwasserstoff der Formel

$$HS - C \equiv N$$

Beispiele für Wirkstoffe der Formel V sind die folgenden:

$$H_{25}-C_{12}-(OC_2H_4)_{9-12}-OH$$

Polydocanol
(XIX)

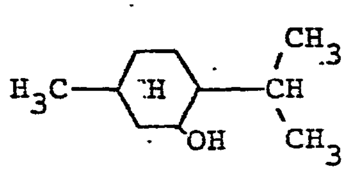

Menthol
(XX)

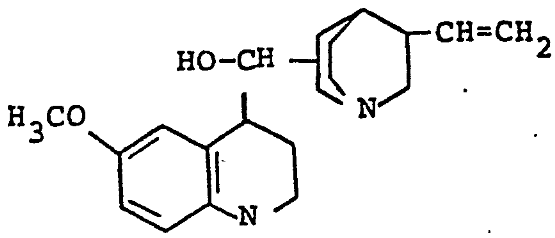

Chinin (XXI)

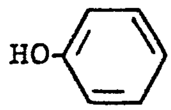

Phenol (XXII)

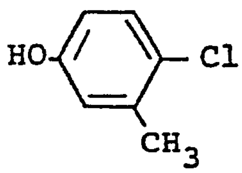

o-Chlor-m-Cresol (XXIII)

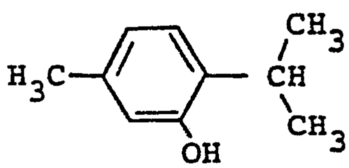

Thymol (XXIV)

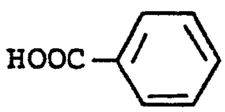

Benzoesäure (XXV)

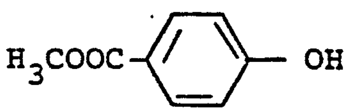

PHB-Methylester (XXVI)

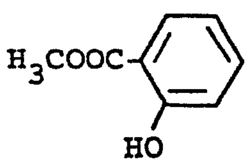

Salicylsäuremethylester ((XXVII)

$HOOC-(CH_2)_8-CH=CH_2$

Undecylsäure (XXVIII)

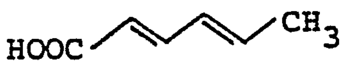

Sorbinsäure (XXIX)

Beispiele für Wirkstoffe der Formel VI sind die folgenden:

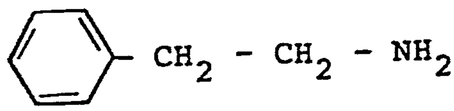

Phenylethylamin (XXX)

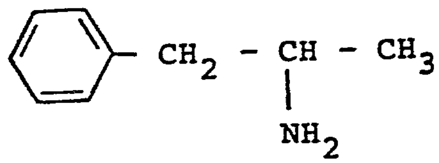

Amphetamin (XXXI)

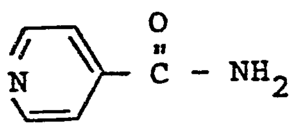

Nicotinamid (XXXII)

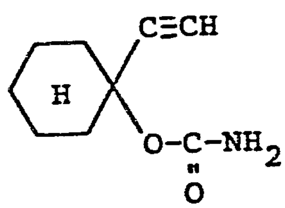

Ethinamat (Valamin®) (XXXIII)

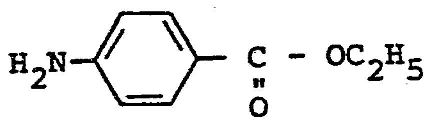

Benzocain (XXXIV)

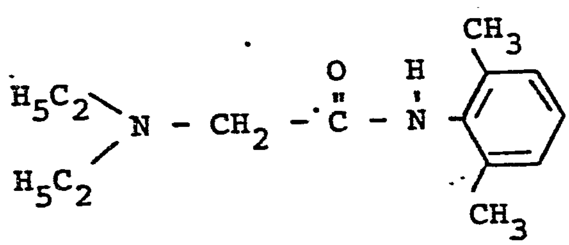

Lidocain (Xylocain®) (XXXV)

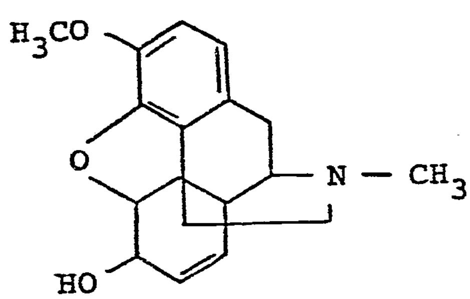

Codein (XXXVI)

Beispiele für Wirkstoffe der Formel VII sind die folgenden:

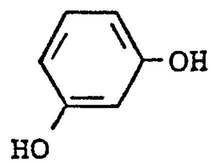

Resorcin (XXXVII)

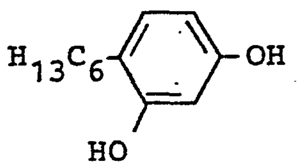

Hexylresorcin (XXXVIII)

$Cl_3C - CH(OH)_2$

Chloralhydrat (XXXIX)

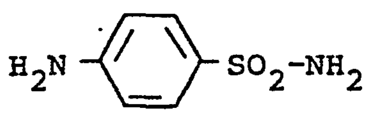

Sulfanilamid (XXXX)

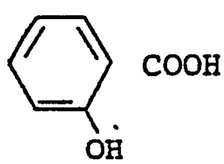

Salicylsäure (XXXXI)

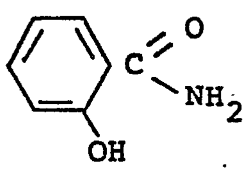

Salicylamid (XXXXII)

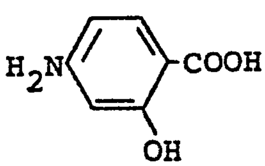

p-Aminosalicylsäure (XXXXIII)

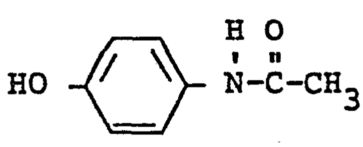

Paracetamol (XXXXIV)

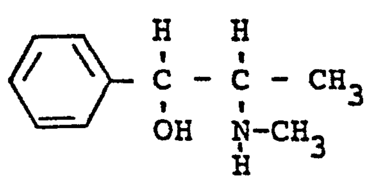

Ephedrin (XXXXV)

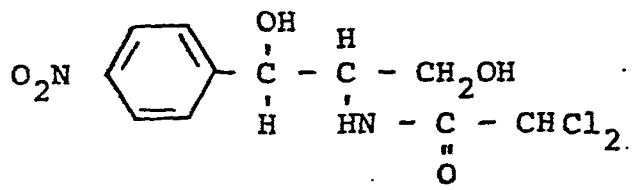 Chloramphenicol (XXXXVI)

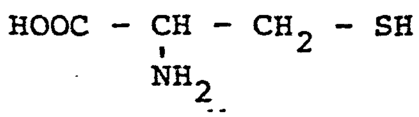 Cystein (XXXXVII)

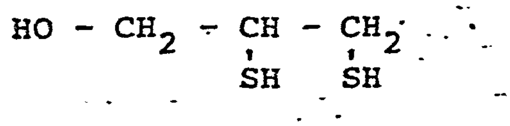 Dimercaprol (BAL) (XXXXVIII)

Besonders bevorzugte erfindungsgemäße Verbindungen sind Verbindungen der folgenden Formeln:

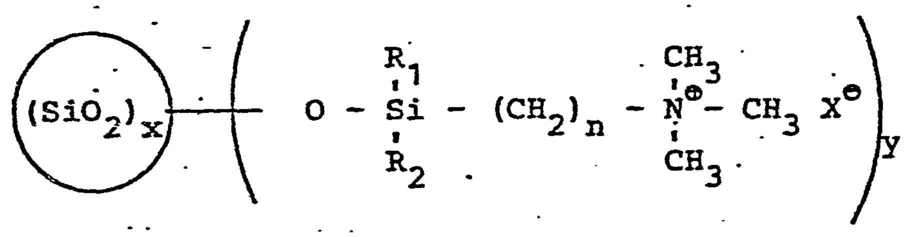 (VIII)

worin $X^{\ominus}$ ein Halogenanion, wie ein Jod-, Chlor- oder Bromanion, oder ein anderes pharmazeutisch annehmbares Anion, wie ein anorganisches oder organisches Anion, bedeutet. Beispiele für pharmazeutisch annehmbare anorganische Anionen sind das Sulfat-, Phosphat- oder Nitratanion, und Beispiele für organische Anionen sind Acetat, Tartrat, Succinat, Lactat und Citrat. In der obigen Formel VIII bedeutet n 1 bis 8, vorzugsweise 3, und $R_1$ und $R_2$ bedeuten eine Methyl- oder Hydroxylgruppe. Besonders bevorzugte Verbindungen sind solche, worin $R_1$ und $R_2$ beide eine Hydroxylgruppe bedeuten. y kann in der obigen Formel 0,1 bis 0,7 mMol/g bedeuten, bevorzugt bedeutet es 0,3 bis 0,5 mMol/g. Eine besonders bevorzugte Verbindung der Formel VIII ist die folgende Verbindung der Formel IX:

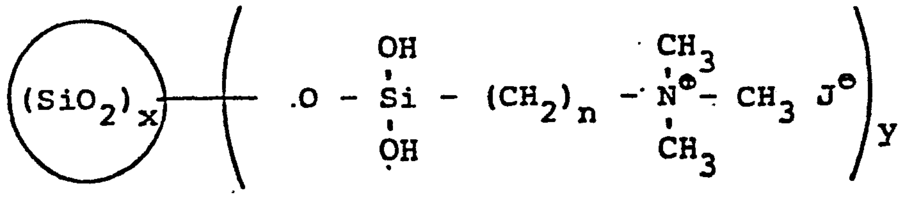 (IX)

worin y 0,1 bis 0,5 mMol/g, bevorzugt 0,2 bis 0,4 mMol/g, bedeutet.

Eine weitere Klasse von bevorzugten Verbindungen sind solche der Formel X

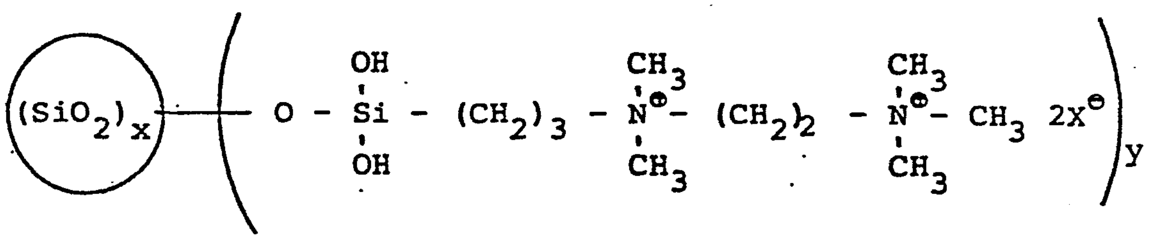

(X)

worin y 0,10 bis 0,5, vorzugsweise 0,2 bis 0,35 und besonders bevorzugt 0,25, mMol/g bedeutet und $X^{\ominus}$ die oben im Zusammenhang mit der Formel VIII gegebene Bedeutung besitzt. In der Verbindung der Formel (X) ist $X^{\ominus}$ besonders bevorzugt Jod.

Eine weitere Klasse von bevorzugten Verbindungen sind solche der Formel XI

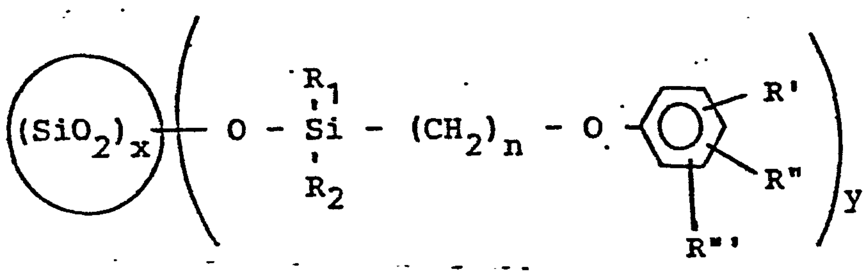 (XI)

worin n 1 bis 8, vorzugsweise 3, bedeutet und R', R" und R"' Wasserstoff oder übliche Substituenten am aromatischen Ring, wie Halogene, beispielsweise Chlor, Fluor, Brom oder

Jod, $C_1$-$C_5$-Alkyl, vorzugsweise Methyl, $C_1$-$C_2$-Alkoxy, vorzugsweise Methoxy, -CHO, -OH, -CN oder $-NH_2$-Gruppen bedeuten und y 0,1 bis 0,5 mMol/g bedeutet.

Eine besonders bevorzugte Verbindung der Formel XI ist die folgende Verbindung der Formel XII

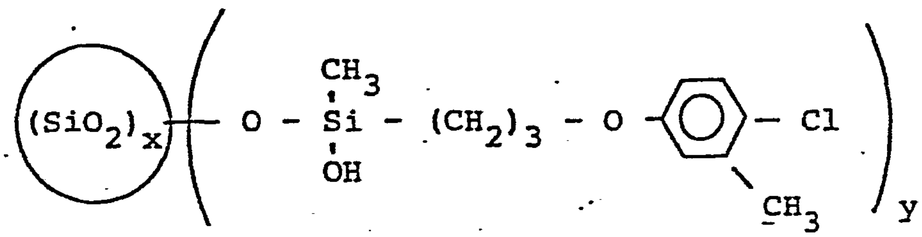

(XII)

worin y 0,1 bis 0,5 mMol/g bedeutet.

Weitere bevorzugte Verbindungen sind die folgenden Verbindungen der Formeln XIII, XIV, XV und XVI:

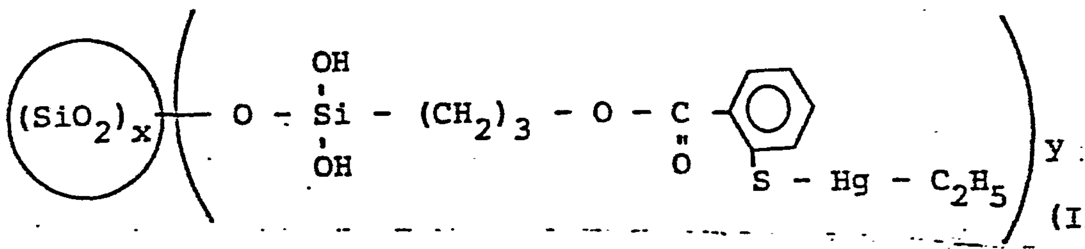

(IIIX)

worin y 0,1 bis 0,5 mMol/g bedeutet,

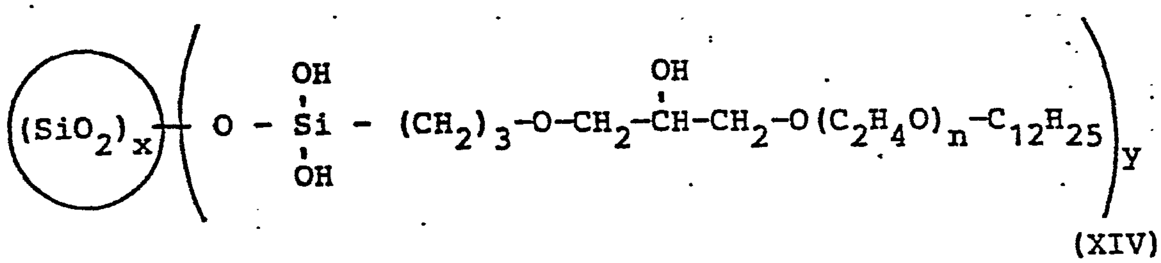

(XIV)

worin n 9 bis 12 und y 0,1 bis 0,4 mMol/g bedeuten,

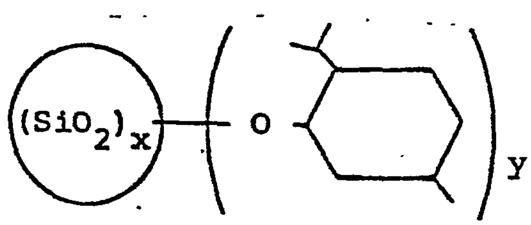

(XV)

worin y 0,1 bis 0,5 mMol/g bedeutet, und

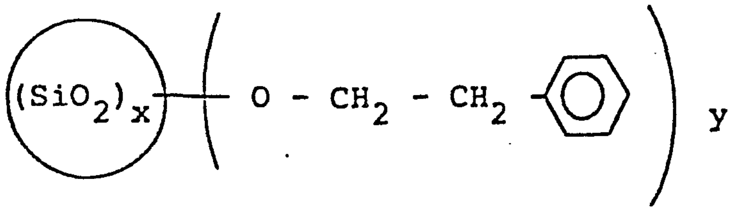

(XVI)

worin y 0,1 bis 0,5 mMol/g bedeutet.

Im folgenden werden die Verfahren zur Herstellung der erfindungsgemäßen Verbindungen näher erläutert.

1) <u>Herstellung von Verbindungen der Formel I</u>

Prinzip des Herstellungsverfahrens:
Unter wasserfreien Bedingungen wird in einem unpolaren organischen Dispersionsmittel, wie beispielsweise Tetrachlorkohlenstoff, 1,2-Dichlorethan, Cyclohexan, Xylol, Toluol oder Gemischen dieser Flüssigkeiten, eine stöchiometrische Menge hochdisperser Kieselsäure mit definierter spezifischer Oberfläche und bekannter Zahl an reaktiven Silanolgruppen dispergiert und mit der Lösung eines reaktiven Alkoxy-, vorzugsweise Ethoxy-, -silans versetzt, das einen reaktiven Spacer oder bereits das chemisch verknüpfte Wirkstoffmolekül trägt.

Im folgenden physikalischen Adsorptionsschritt, der bei Temperaturen zwischen -10°C und +30°C, vorzugsweise bei Raumtemperatur (20°C), erfolgt, wird ein definiertes Adsorptionsgleichgewicht eingestellt. Ausgehend von der Kenntnis der Adsorptionseigenschaften kann in einem weiten Bereich bis zur maximalen Belegung der Oberfläche Wirkstoff gebunden werden. Der maximale Belegungswert ergibt sich einmal aus dem Platzbedarf der Reaktanden und ist andererseits durch die Zahl 4 OH-Gruppen/1 $nm^2$ am Träger begrenzt. (R.K. Iler, "The Chemistry of Silica", John Wiley & sons, New York, Chichester, Toronto 1979, Seiten 633 ff.)

Das Sorbat wird anschließend durch Zentrifugieren und Dekantieren von der Gleichgewichtsflüssigkeit entfernt oder, falls im Adsorptionsgleichgewicht die in Lösung befindliche Menge vernachlässigbar klein ist, das Dispersionsmittel im Vakuum entfernt. Im folgenden Schritt wird die Chemisorption durch Erhöhen der Temperatur auf 80 bis 220°C, vorzugsweise auf 150°C, eingeleitet, wobei unter Abspaltung von Ethanol bei den Ethoxysilanen bzw. HCl bei Chlorsilanen eine Verankerung über die stabile Si-O-C-Brücke erfolgt.

Als Beispiel für beide Schritte sind nachfolgend jeweils ein mit dem Wirkstoff bereits verankertes Ethoxysilan mit der Spacer-Gruppe Z bzw. ein Chlorsilan mit der Spacer-Gruppe Z aufgeführt.

<u>Physikalische Sorption:</u>

a) <u>Ethoxysilane</u>

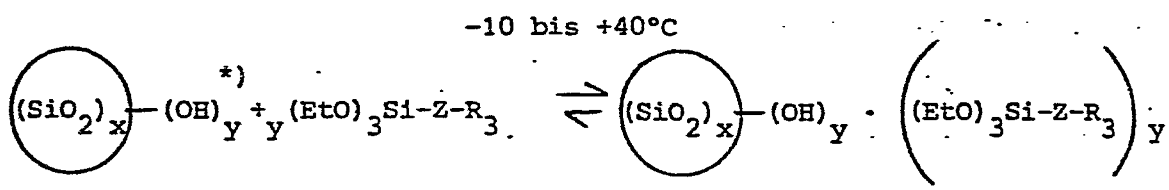


b) <u>Chlorsilane</u>

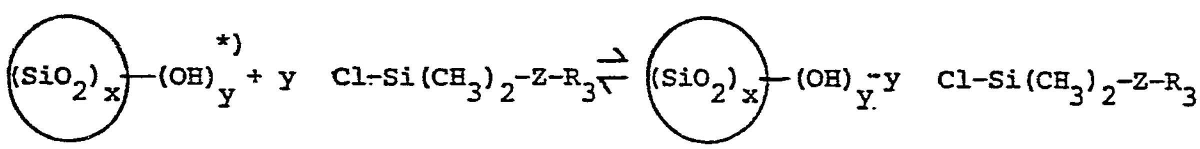

<u>Chemisorption:</u>

a)

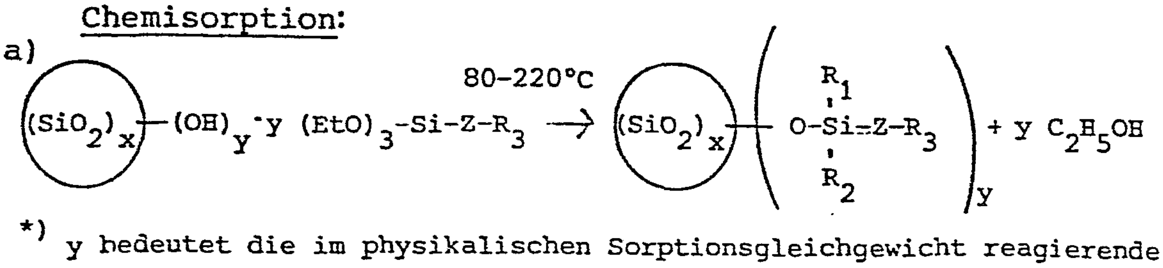


*) y bedeutet die im physikalischen Sorptionsgleichgewicht reagierende Menge an SiOH-Gruppen und bedeutet ≦ der maximalen Menge an reaktionsfähigen Silanolgruppen von 6,6 $10^{-6}$ Mol $m^{-2}$.

b)

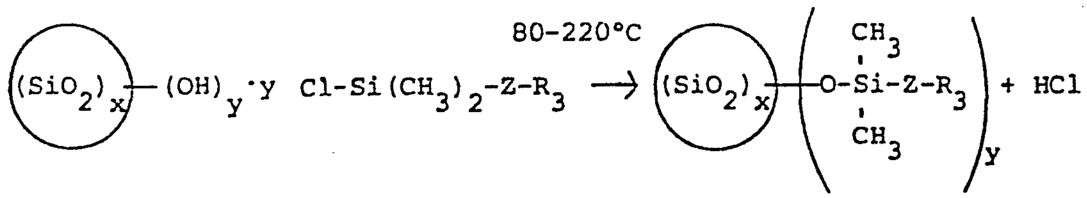


Das während des Chemisorptionsschritts freiwerdende Ethanol bzw. HCl wird durch Destillation aus dem Reaktionsgleichgewicht laufend entfernt. Bezüglich der Verankerung mit der Oberfläche laufen etwa zu gleichen Teilen mono- und bifunktionelle Verankerungen der Ethoxysilane und Chlorsilane mit mehr als zwei Ethoxy- bzw. Chlorgruppen pro Molekül mit der Oberfläche ab. Hierbei ist von einer etwa gleichen Verteilung beider Verknüpfungsarten auszugehen, wobei zunächst die Kapazität der Oberfläche beschränkt erscheint. Da jedoch der Platzbedarf der organischen Reste wesentlich größer ist als der durchschnittliche Platzbedarf der Silanolgruppen an der Oberfläche bei max. 4 OH/$nm^2$ (0,25 $nm^2$/Silanolgruppe, mindestens 0,40 $nm^2$/ für organische Gruppen), wird die Aufnahmekapazität bezüglich der organischen Reste kaum verändert: Man kann davon ausgehen, daß maximal 2,5 organische Reste 1 $nm^2$ eingeführt werden können.

Durch die gewählte Art der Verankerung ist es möglich – im Gegensatz zur Reaktion aus der Gasphase –, eine sehr gleichmäßige Verteilung der reaktiven Gruppen an der Oberfläche zu erreichen. Während des Einstellens des physikalischen Adsorptionsgleichgewichts sind alle Teilchen bwz. ihre Oberflächen für das Reagenz gleichmäßig zugänglich.

Erfahrungsgemäß werden sehr gleichmäßig belegte Präparate erhalten, wenn bei der anschließenden Chemisorption der Siedepunkt des jeweiligen Reagenz nicht überschritten wird.

0101027

Bei den Reaktionen wird die Verankerung mittels Ethoxysilanen bevorzugt, da hierbei der weniger aggressive Ethanol abgespalten wird, während bei den Chlorsilanen durch das entwickelte HCl eher mit unerwünschten Nebenreaktionen zu rechnen ist.

Für den gesamten Verlauf der Verankerung der Chlorsilane bzw. der Ethoxysilane wurden drei Verfahrensvarianten entwickelt, die folgende Gesichtspunkte berücksichtigen:

1. Reaktivität der zu verankernden Wirksubstanz, der Spacer-Gruppe und des $SiO_2$
2. Reinigung der Produkte
3. Charakterisierung der Zwischen- und Endprodukte

Variante 1:

Der Wirkstoff wird zunächst an das Ethoxysilan oder Chlorsilan über eine reaktive Gruppe, gegebenenfalls mit Spacer, chemisch gebunden und diese Verbindung dann mit der $SiO_2$-Oberfläche umgesetzt. Beispiele für Spacer der Formel VIII sind die folgenden:

$$Cl_2SiH - (CH_2)_3 - Cl$$

X = EtO oder Cl oder (H, $Cl_2$)

$$X_3Si - (CH_2)_3 - O-\overset{O}{\overset{\|}{C}}-CH_3$$

$$X_3Si - (CH_2)_3 - OH$$

$$X_3Si - (CH_2)_3 - NH_2$$

$$X_3Si - (CH_2)_n - NH_2 \qquad n = 1 \text{ bis } 9$$

$X_3Si - (CH_2)_3 - Cl$

$X_3Si - (CH_2)_n - Cl$ $n = 1$ bis 9

$X_3Si - (CH_2)_3 - NH - (CH_2)_3 - NH_2$

$X_3Si - [(CH_2)_3-O]_n - CH_2 - \overbrace{CH - CH_2}^{O}$

Beispiel für ein grenzflächenverankertes Polydocanol: Im ersten Reaktionsschritt wird Triethoxysilan mit Alkylglycidylepoxid in Gegenwart von katalytisch wirkender Hexachloroplatinsäure in Toluol bei 120°C umgesetzt, um einen reaktiven Spacer (Epoxid) mit dem Ethoxysilan zu verknüpfen.

1) $(EtO)_3\ Si\text{-}H + H_2C{=}C\text{-}CH\text{-}CH_2\text{-}O\text{-}CH_2\text{-}\overbrace{CH\text{-}CH_2}^{O}$ $\xrightarrow[\text{platinsäure}]{\text{Hexachloro-}}$

Triethoxysilan   Alkylglycidylepoxid   Toluol 120°C

$(EtO)_3\text{-}Si\text{-}(CH_2)_3\text{-}O\text{-}CH_2\text{-}\overbrace{CH\text{-}CH_2}^{O}$

I'

2) Das mit dem Triethoxysilan verknüpfte Epoxid wird anschließend mit Polydocanol verethert:

$I' + HO - (C_2H_4O)_{9-12}- C_{12}H_{25}$ $\xrightarrow[\text{Raumtemperatur}]{\text{Rühren}}$

Polydocanol

$(EtO)_3\text{-}Si\text{-}(CH_2)_3\text{-}O\text{-}CH_2\text{-}\underset{}{\overset{OH}{CH}}\text{-}CH_2\text{-}O(C_2H_4O)_{9-12}\text{-}C_{12}H_{25}$

II'

3) In einem dritten Reaktionsschritt erfolgt dann die Verknüpfung mit der Oberfläche des Trägermaterials:

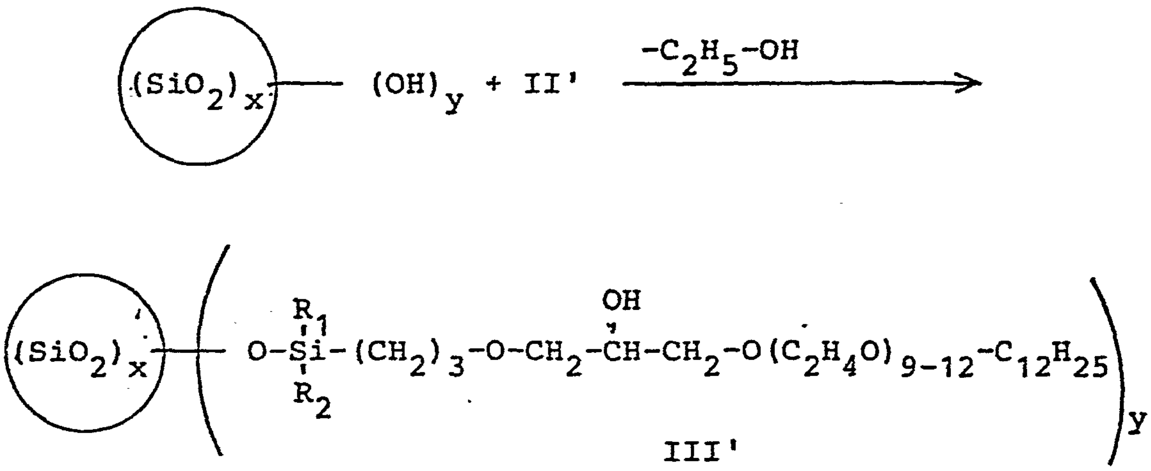


III'

4) Nach anschließendem Entfernen der flüchtigen Reaktionsprodukte (Ethanol im Vakuum) wird das Produkt gegen Wasser dialysiert, wobei noch vorhandene Ethoxygruppen hydrolytisch abgespalten werden:

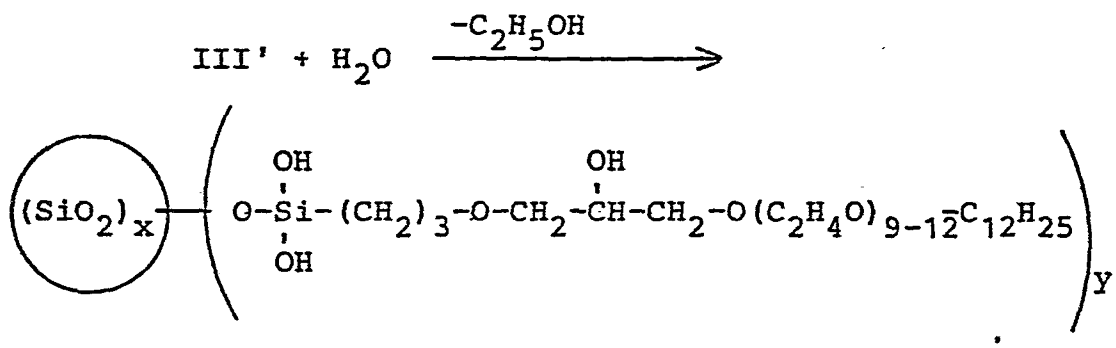


<u>Variante 2</u>:

Das Ethoxysilan bzw. Chlorsilan mit reaktiver Gruppe, gegebenenfalls mit Spacer, wird zuerst an der Siliciumdioxidoberfläche verankert und dann in einem weiteren Schritt über die reaktive Gruppe mit dem Arzneistoff verknüpft.

<u>Beispiel: Herstellung von fest gebundenem p-Chlor-m-cresol</u>

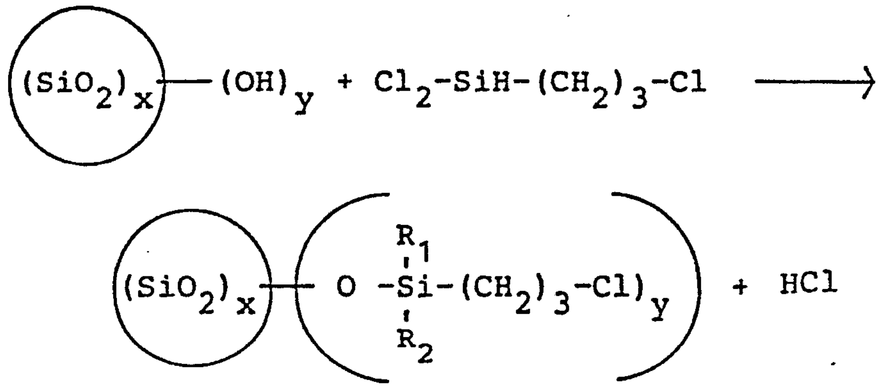

III

Im ersten Schritt wird die Oberfläche mit p-Chlorpropyldichlorsilan unter wasserfreien Bedingungen in 1,2-Dichlorethan nach Einstellung eines physikalischen Adsorptionsgleichgewichts bei 120°C unter HCl-Abspaltung verknüpft. Nach Entfernung flüchtiger Reaktionsprodukte (HCl) erfolgt im zweiten Schritt die Reaktion des Phenols mit dem Alkylchlorid:

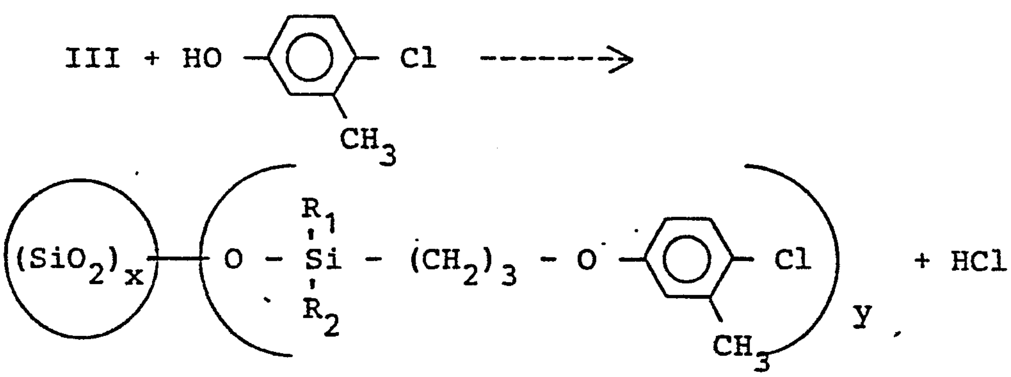

IV

wobei ein Phenolether (Verbindung IV) entsteht. Diese Reaktion findet bei 200°C, 4 h statt. Beim folgenden Reinigungsprozeß, Dialyse gegen Wasser, werden die mit $R_1$ und $R_2$ bezeichneten Silan- bzw. Chlorsilangruppen zu Hydroxylgruppen hydrolisiert (siehe Formel V).

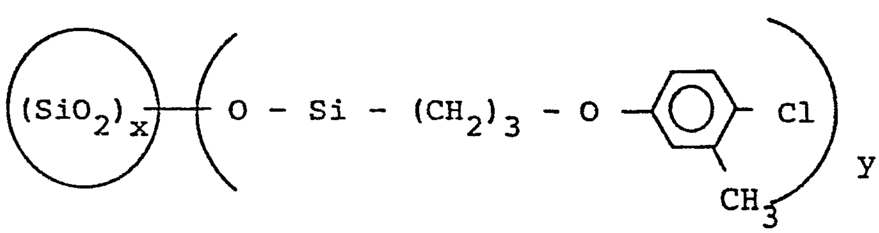

V

Beispiel: 2-Ethylmercurithiobenzoesäureester

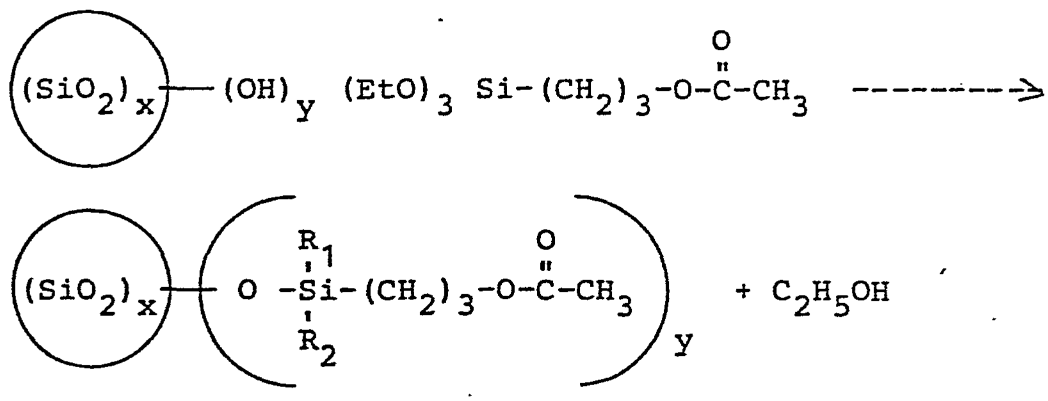

VI

Kieselsäure wird zunächst mit Acetylpropyltriethoxysilan umgesetzt (bei 170°C Chemisorption, in Xylol), danach wird die Acetylschutzgruppe abgespalten: siehe Formelbild

Verseifung der Acetylgruppe:

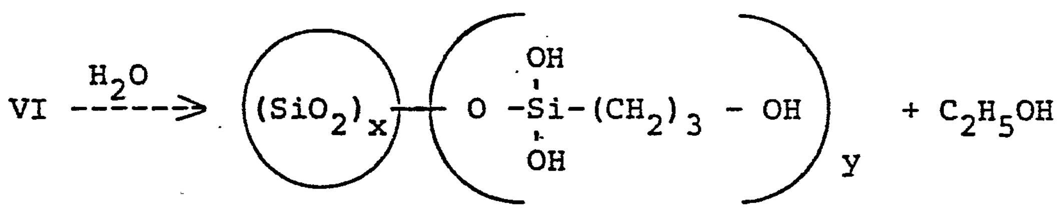


VII

Schließlich wird in einem weiteren Schritt Thiomersalsäure mit dem Alkohol an der Oberfläche (VII) verestert. Dies erfolgt in Xyloldispersion.

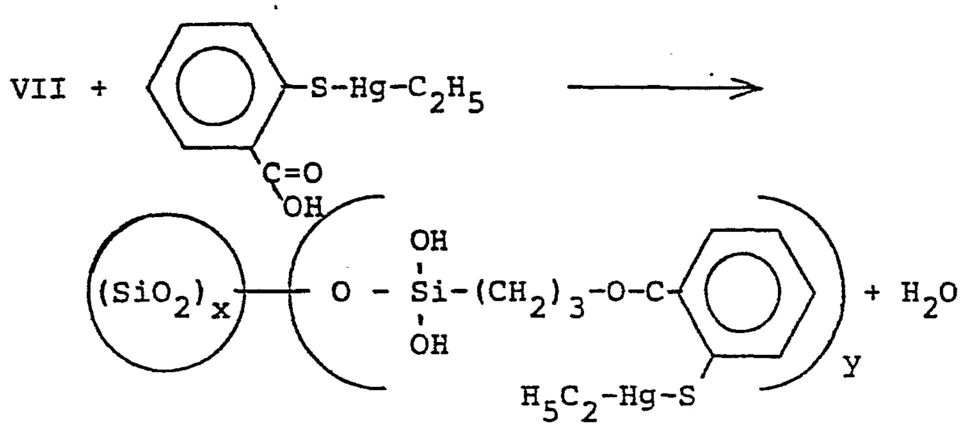

Variante 3:

Das Ethoxysilan oder Chlorsilan mit reaktiver Gruppe (Spacer) wird zuerst mit einer Vorstufe des Wirkstoffs verknüpft und dann in einem weiteren Schritt an der Oberfläche verankert. Die verankerte Vorstufe wird dann - gegebenenfalls in mehreren Stufen - zum Wirkstoffmolekül umgesetzt.

Beispiel: Quartäre Ammoniumverbindungen

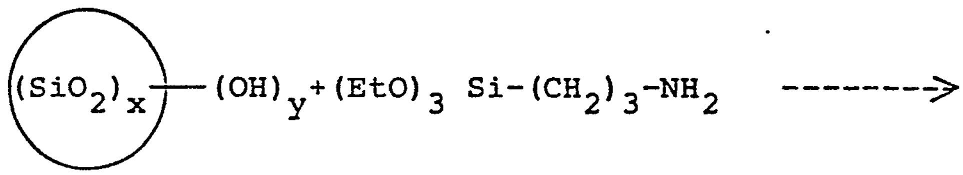

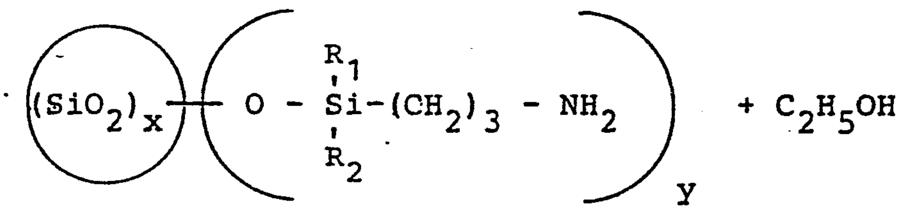

(X)

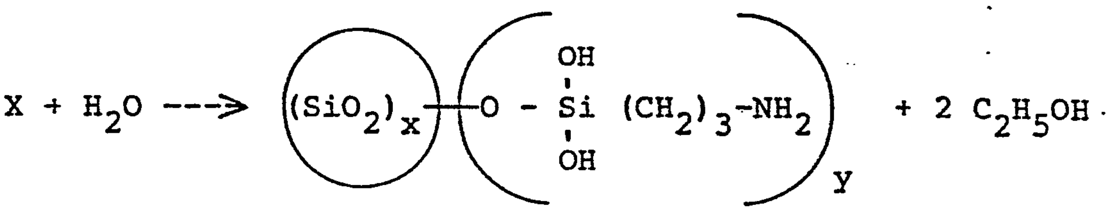

XI

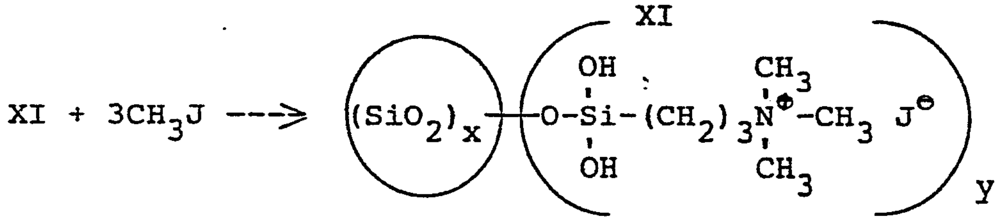

XII

Bei diesem Beispiel besitzt das 3-Aminopropyltriethoxysilan bereits die notwendige funktionelle Gruppe zum Aufbau der N-Verbindung. Nach der Verankerung erfolgt die Methylierung mit Methyljodid zur quartären Verbindung, wobei als Gegenion zunächst Jod nach Ionenaustausch (Behandlung mit Natriumchlorid) Chloridionen eingeführt werden.

Gegenüberstellung der verschiedenen Varianten:

Variante 1 ist vorzuziehen, wenn die Bedingungen während der Umsetzung mit der Trägeroberfläche ohne Einfluß auf die Stabilität und chemische Natur des Wirkstoffs sind. Dabei ist es möglich, in üblicher Weise Vor- und Zwischenstufen herzustellen, zu reinigen und zu charakterisieren.

Variante 2 ist dann vorzuziehen, wenn der Wirkstoff oder die Bindung des Wirkstoffs an den Spacer durch die Reaktionsbedingungen bei der Verknüpfung mit dem Träger beeinfluß wird (z.B. Verseifung, Veresterung).

Variante 3 ist vorzuziehen, wenn sich Vorstufen des Wirkstoffs an der Oberfläche leichter einführen lassen, zum Beispiel aus sterischen Gründen.

Reinigung der Präparate:

Die hergestellten Verbindungen werden nach Entfernung flüchtiger Reaktionsprodukte im Vakuum durch Waschen mit organischen Flüssigkeiten gereinigt. Die Reinigung wird anschließend durch Dialyse gegen Wasser vervollständigt. Bei diesem Schritt können gegebenenfalls noch vorhandene Ethoxy- oder Chlorsilangruppen zu Hydroxylgruppen reagieren (Substituenten $R_1$ und $R_2$ in Formel I nach Anspruch 1). Die Dialyse gegen Wasser wird solange fortgesetzt, bis im eingeengten Dialysat keine organischen Gruppen mit üblichen analytischen Verfahren nachweisbar sind.

Eigenschaften:

Die Produkte fallen nach dem Trocknen der gereinigten Präparate als kolloiddispergierbare Pulver an und zeigen entsprechend den chemischen Eigenschaften der an der Oberfläche verankerten Gruppen und der Belegungsdichte unterschiedliche Benetzungsfähigkeit: Bei dichter Belegung mit vorwiegend hydrophoben Gruppen sind die Präparate schlecht benetzbar. Reduzierung der Belegungsdichte auf etwa 20% der verfügbaren reaktionsfähigen Silanolgruppen an der Oberfläche führt selbst bei langen Kohlenwasserstoffketten (C 8) zu einer guten Benetzbarkeit, so daß die Sorbate leicht in Wasser dispergiert werden können.
Damit ist ihre weitere Verarbeitung zu Pulvern, Pudern, Granulaten, Kapseln, Tabletten, Dragees, Salben, Suppositorien und Suspensionen ohne weiteres möglich.

Normierung:

Qualitativ: Nachweis der funktionellen Gruppen durch IR- und UV-Spektrometrie in situ (Suspensionstechnik, Preßlinge)

Quantitativ: Elementaranalyse auf Kohlenstoff, gegebenenfalls Stickstoff und Schwefel

Quantitative IR-, ggf. UV-Spektrometrie (auch nach Auflösen der Matrix in Lauge oder mit Fluorwasserstoff)

Bei Zwischenprodukten: NMR und Massenspektrometrie

Funktionsnachweise: z.B. Bestimmung der Ionenaustauschkapazität, der mikrobiologischen Hemmwirkung, der lokalanästhetischen Wirkung etc.

2. Herstellung von Verbindungen der Formel II:

Prinzip des Herstellungsverfahrens:

Unter wasserfreien Bedingungen wird in an sich bekannter Weise die Oberfläche von $SiO_2$ mit Thionylchlorid oder einem anderen chlorierenden Agens chloriert. Das entstehende Produkt trägt an seiner Oberfläche anstelle der ursprünglichen Silanolgruppen Chlor, so daß formal ein Chlorid der Kieselsäure vorliegt. Diese Oberfläche ist äußerst reaktionsfähig und bindet Protonen liefernde Substanzen unter HCl-Abspaltung, wie zum Beispiel Wasser, Alkohole, Säuren, Amine, Amide, Lactame oder Phenole, wobei eine labile SiOC- bzw. SiNC-Bindung oder SiSC-Bindung zwischen Oberfläche und organischem Rest gebildet wird.

Steuerung der Herstellung:

Die maximale Belegbarkeit der Oberfläche wird zunächst durch den Grad der Chlorierung, vor allem aber durch Sperrigkeit (Platzbedarf) und Reaktionsfähigkeit der zu verankernden Gruppen bestimmt. Bei der Chlorierung mit Thionylchlorid in Benzol nach Boehm und Janßen wird ein maximal erreichbarer Austausch der Silanolgruppen durch Chlor erreicht. Die Belegungsdichte von 3 bis 4 Chlorgruppen/1 $nm^2$ an der Kieselsäureoberfläche gewährleistet selbst bei kleinen organischen Gruppen eine hinreichende Dichte zu einer vollständigen Belegung. Organische Gruppen haben dabei mindestens einen Platzbedarf von 0,40 $nm^2$, so daß maximal 2,5 organische Reste, in den meisten Fällen weniger wegen des höheren Platzbedarfs, gebunden werden können. Nicht umgesetztes Chlorid der Oberfläche wird beim ersten Kontakt mit Wasser in Form von HCl abgespalten. Daher ist es zweckmäßig, die vollständige Chlorierung der Oberfläche zu vermeiden und die Präparate im ersten Reaktionsschritt so einzustellen, daß eine stöchiometrische Umsetzung gewährleistet ist. Da-

mit kann einerseits ausreichend Arzneistoff chemisorbiert, auf der anderen Seite während der Anwendung die Abspaltung von HCl vermieden werden. Die Steuerung der Chlorierung ist ohne weiteres durch die Reaktionsbedingungen (Mengenverhältnis beim Ansatz, Temperatur und Zeitdauer) in bekannter Weise möglich. Die eigentliche Umsetzung mit dem Wirkstoff wird, ähnlich wie bei Verfahren nach Verbindungen der Formel I zunächst durch Einstellen eines physikalischen Adsorptionsgleichgewichts gesteuert. Hierbei wird eine gleichmäßige Verteilung des Wirkstoffs an der Oberfläche angestrebt. Die weitere Verarbeitung erfolgt analog, wobei allerdings das Auswaschen der Produkte unter wasserfreien Bedingungen erfolgen muß. Für die Charakterisierung der Produkte gelten ähnliche Gesichtspunkte wie oben beschrieben.

Die folgenden Beispiele erläutern die Erfindung.

 

## Herstellung der Präparate

### Wassergehalt

Wegen der Hydrolyseempfindlichkeit der verwendeten Silane bzw. der chlorierten Oberfläche des $SiO_2$ und zur besseren Überschaubarkeit der Reaktionswege an der $SiO_2$-Oberfläche wird unter wasserfreien Bedingungen gearbeitet.

### Ausgangssubstanzen

Lösungsmittel werden über Molekularsiebperlen (3 Å) aufbewahrt, feste Reagenzien im Trockenschrank bei 110°C getrocknet oder 24 h über $P_2O_5$ im Exsiccator gelagert. Die Kieselsäure (Aerosil 200, Degussa, spezifische Oberfläche 234 $m^2/g$, BET mit $N_2$) wird 5 h bei 200°C getrocknet.

### Reaktionsbedingungen

Abfüll-, Wäge und Mischvorgänge werden in trockenem $N_2$ in einer Glove-Box vorgenommen. Die Umsetzungen werden unter starkem Rühren durchgeführt (heterogene Reaktion).

## Beispiel 1

3-Trimethylammonium-n-propylsilyl-Chemisorbat

28,50 g (≙ 0,04 Mol Silanolgruppen) Aerosil 200 werden mit 16 g (0,07 Mol) 3-Aminopropyltriethoxysilan unter ständigem Rühren 1 h bei Raumtemperatur gemischt und dann 8 h in Xylol am Rückfluß erhitzt. Danach wird abdestilliert und der Rückstand 4 h bei 473°K im Vakuum (Enddruck 0,1 Pa) ausgeheizt, um flüchtige Reaktionsprodukte zu entfernen.

10,0 g des Rückstands (3-Amino-n-propylsilyl-Chemisorbat, entspr. ca. 0,015 Mol) werden mit 7 g Methyljodid (0,05 Mol) in Acetonitril unter Rühren 1 h gemischt und dann 2 h am Rückfluß erhitzt. Danach wird das Lösungsmittel abdestilliert und der Rückstand 4 h bei 473°K (Enddruck 0,1 Pa) ausgeheizt. Das gegen Wasser dialysierte Produkt wies einen Gehalt von 0,076 mMol $g^{-1}$ organische Gruppen auf (C-Gehalt, Austauschkapazität). Escherichia coli wird von 0,1% des Produkts vollständig inhibiert (Warburg-Test).

BAD ORIGINAL

B e i s p i e l 2

p-Chlor-m-cresol-Chemisorbat

28,50 g ($\hat{=}$ 0,04 Mol Silanolgruppen) Aerosil 200 werden mit 5 g (0,02 Mol) 3-Chlorpropylmethyldichlorsilan unter ständigem Rühren 8 h in 1,2-Dichlorethan am Rückfluß erhitzt. Danach wird abdestilliert und der Rückstand 4 h bei 393$^{O}$K im Vakuum (Enddruck 0,1 Pa) ausgeheizt, um flüchtige Reaktionsprodukte zu entfernen.

Danach wird der Rückstand mit 3,15 g p-Chlor-m-cresol (0,02 Mol) unter ständigem Rühren 8 h in 1,2-Dichlorethan am Rückfluß erhitzt. Anschließend wird das Lösungsmittel abdestilliert und der Rückstand 4 h bei 473°K im Vakuum (Enddruck 0,1 Pa) ausgeheizt, um flüchtige Reaktionsprodukte zu entfernen. Das gegen Wasser dialysierte Produkt weist einen Gehalt von 0,64 mMol/g auf. Es liegt als weißes, schwer benetzbares Pulver vor.

B e i s p i e l 3

Thiomersal-Chemisorbat

28,50 g ($\hat{=}$ 0,04 Mol Silanolgruppen) Aerosil 200 werden mit 15 g (0,06 Mol) o-Acetyl-n-propyl-triethoxysilan unter ständigem Rühren 8 h in Xylol am Rückfluß erhitzt. Danach wird abdestilliert und der Rückstand 4 h bei 473$^{O}$K im Vakuum (Enddruck 0,1 Pa) ausgeheizt, um flüchtige Reaktionsprodukte zu entfernen.

Nach Abdestillieren des Lösungsmittels und Trocknen bei 473$^{O}$K während 4 h bei 0,1 Pa wird mit 100 ml 12% wäßriger Ammoniaklösung 1 h am Rückfluß erhitzt. Nach Abdestillieren der flüssigen Phase wird bei 473$^{O}$K und 0,1 Pa ausgeheizt (4 h).

7, 42 g ($\hat{=}$ unter 0,01 Mol) des so hergestellten Chemisorbats werden mit 4,35 g (0,01 Mol) Thiomersal®-Säure unter ständigem Rühren 8 h in 100 ml Xylol am Rückfluß erhitzt.

Danach wird abdestilliert und der Rückstand zum Abtrennen der löslichen Nebenprodukte mit 100 ml Dichlorethan und nach dem Trocknen mit 200 ml $H_2O$ extrahiert und mittels Dialyse gereinigt. Das Produkt weist einen Gehalt von 0,58 mMol/g organische Gruppen auf. Staphylococcus aureus, Streptococcus faecalis, Proteus mirabilis werden im Warburg-Test von 0,1% Sorbat vollständig inhibiert.

### B e i s p i e l 4

Phenetol-Chemisorbat

30 g (≙ 0,042 Mol Silanolgruppen) Aerosil 200 werden mit 60 g (0,5 Mol) Thionylchlorid unter ständigem Rühren 8 h in 240 g Benzol am Rückfluß erhitzt. Danach wird abdestilliert und der Rückstand 4 h bei 473°K im Vakuum (Enddruck 0,1 Pa) ausgeheizt, um flüchtige Reaktionsprodukte zu entfernen.

25 g des Rückstands (oberflächengebundenes Kieselsäurechlorid, entspr. 0,04 Mol) werden mit 50 g Phenetol (0,4 Mol) 8 h in 240 g Benzol am Rückfluß erhitzt. Danach wird das Lösungsmittel abdestilliert und der Rückfluß 4 h bei 473°K im Vakuum (Enddruck 0,1 Pa) ausgeheizt. (Entfernung flüchtiger Reaktionsprodukte). Eigenschaften: weißes Pulver mit 0,65 Mol Phenolgruppen; Phenetol wird innerhalb einer Stunde abgespalten.

### B e i s p i e l 5

Menthol-Chemisorbat

30 g (≙ 0,042 Mol Silanolgruppen) Aerosil 200 werden, wie bei Phenetol beschrieben (Beispiel 4), mit Thionylchlorid zum Oberflächen-Kieselsäurechlorid umgesetzt und gereinigt.

25,6 g des Rückstands (oberflächengebundenes Kieselsäurechlorid, entspr. ca. 0,04 Mol) werden mit 6,5 g Menthol (0,04 Mol) 8 h in 240 g Benzol am Rückfluß erhitzt.

Danach wird das Lösungsmittel abdestilliert und der Rückstand 4 h bei 473°K im Vakuum (Enddruck 0,1 Pa) ausgeheizt. Eigenschaften: weißes Pulver, das bei Kontakt mit Wasser intensiv nach Menthol riecht. Gehalt an chemisorbiertem Menthol 0,67 $mMol/g^{-1}$. In Wasser wird das chemisorbierte Menthol innerhalb einer Stunde vollständig abgegeben.

B e i s p i e l 6

N(Trimethylammoniumethyl)-dimethylammoniumpropyl-Chemisorbat (28,50 g) auf entsprechend 0,04 Mol Silanolgruppen Aerosil 200 werden mit 6 g N(-ß-Aminoethyl)γ-aminopropyl-trimethoxy-silan unter ständigem Rühren 1 h bei Raumtemperatur gemischt und dann 8 h in Xylol am Rückfluß, 4 h bei 473°K im Vakuum (Enddruck 0,1 Pa) ausgeheizt, um flüchtige Reaktionsprodukte zu entfernen.

20,0 g des Rückstands (Zwischenprodukt) werden mit 13 g Methyljodid in Acetonitril unter Rühren 1 h gemischt und dann 2 h am Rückfluß erhitzt. Danach wird das Lösungsmittel abdestilliert und der Rückstand 4 h bei 473°K (Restdruck 0,1 Pa) ausgeheizt. Das gegen Wasser dialysierte Produkt wies einen Gehalt von 0,057 mMol/g organische Gruppen auf (C-Gehalt). Die Austauschkapazität betrug 1,15 mval/g. Escherichia coli wird im Warburg-Test von 0,5% der Substanz vollständig inhibiert.

B e i s p i e l 7

Dodecanol-Chemisorbat

10 g Alkylglycidylepoxid wurden mit 8,7 g Triethxoysilan in Toluol gemischt und bei 120°C in Gegenwart von 100 ppm Hexachloroplatinsäure umgesetzt. Die Reinigung des Ethoxysilans erfolgte danach durch Destillation bei 3 mm Hg-Säule und einer Temperatur von 123°C.

Zu 20 g des Zwischenprodukts wurden 11 g Polydocanol langsam zugemischt und bei maximal 110°C umgesetzt. Das Reaktionsprodukt (15 g) wurde anschließend mit 11 g Aerosil 200 in Xyloldispersion intensiv gemischt und bei 120°C zur Reaktion gebracht. Nach Reinigung des Endprodukts im Vakuum und anschließender Dialyse gegen Wasser wurde ein weißes pulverförmiges Produkt mit schwachem Geruch erhalten. Berechnet auf Polydocanol, wurde aus der C-Analyse ein Gehalt von 0,78 mMol/g berechnet. Das Produkt hat anästhesierende Wirkung auf die Zungen- und Mundschleimhaut.

PATENTANSPRÜCHE

1. An Siliciumdioxid als Träger gebundene Wirkstoffe der folgenden Formeln I, II oder III

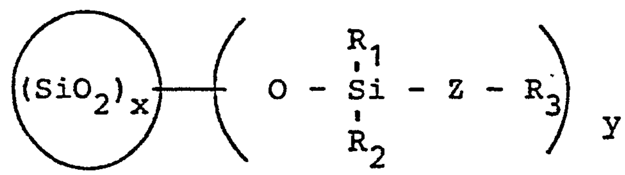

(I)

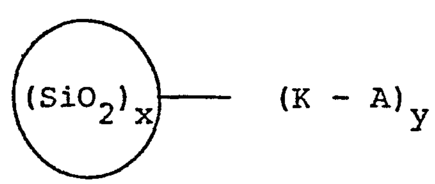

(II)

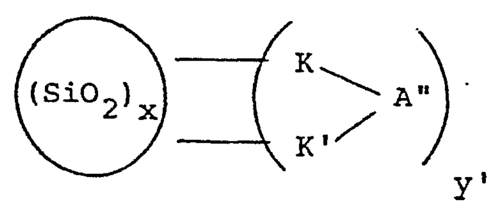

(III)

worin $(SiO_2)_x$ eine Menge hochdisperser Teilchen bedeutet, y und y' die pro Menge oder Oberflächeneinheit des Trägers verankerten Gruppen in Mol bedeuten (mMol/g oder mMol/m²), y im Bereich von 0,1 bis 1 und y' im Bereich von 0,1 bis 0,5 liegen, Z $-(CH_2)_n-$, worin n für 0 bis 18 steht, oder einen Rest der Formeln

$$-[(CH_2)_3-O]_{n'}, \quad -CH_2-\underset{OH}{CH}-CH_2-$$

oder

$$-HN-(CH_2)_3-NH-$$

bedeutet, worin n' für 1 bis 3 steht,

$R_1$ und $R_2$ gleich oder verschieden sein können und eine Methyl- oder eine Hydroxylgruppe bedeuten,

$R_3$ den einwertigen Rest eines Wirkstoffs bedeutet, mit der Maßgabe, daß, wenn Z eine Alkylengruppe bedeutet, in der n für 0 steht, der Wirkstoff unmittelbar mit einem seiner Kohlenstoffatome mit dem Siliciumatom verknüpft ist,

K und K' O, S oder >N-A' bedeuten, wobei in der Formel III die beiden Substituenten K und K' gleich oder unterschiedlich sein können,

A den einwertigen Rest eines Wirkstoffs der Formeln IV, V oder VI

$$H-S-A \qquad (IV)$$

$$H-O-A \qquad (V)$$

$$H-\underset{A'}{N}-A \qquad (VI)$$

bedeutet,

wobei A' ein Wasserstoffatom bedeutet oder wobei in der Formel VI A und A' zusammen mit dem Stickstoffatom, an das sie gebunden sind, den Rest eines Wirkstoffmoleküls bedeuten,

A" den zweiwertigen Rest eines Wirkstoffs der Formel VII

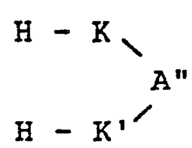

(VII)

bedeutet,
und wobei bei der Verankerung mit dem Träger bei den Wirkstoffen der Formel IV oder VI ein Wasserstoffatom durch den Träger ersetzt wurde und bei dem Wirkstoff der Formel VII zwei Wasserstoffatome durch den Träger ersetzt wurden.

2. Verbindungen nach Anspruch 1 der Formel VIII

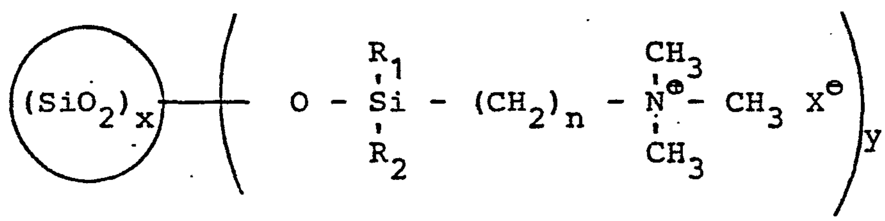

(VIII)

worin $X^{\ominus}$ ein Halogenanion, wie ein Jod-, Chlor- oder Bromanion, oder ein anderes pharmazeutisch annehmbares Anion, n 1 bis 9 , $R_1$ und $R_2$ eine Methyl- oder Hydroxylgruppe und y 0,1 bis 0,7 mMol/g bedeuten.

3. Verbindungen nach Anspruch 2 der Formel IX

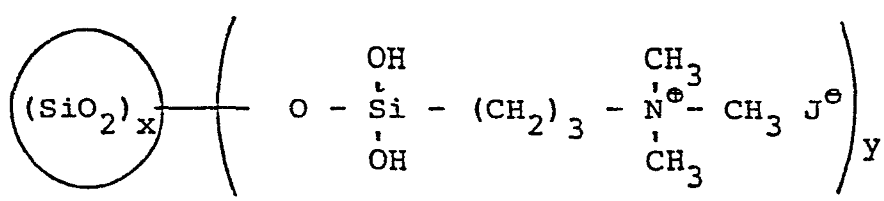

(IX)

worin y 0,1 bis 0,5 mMol/g bedeutet.

4. Verbindungen nach Anspruch 1 der Formel (X)

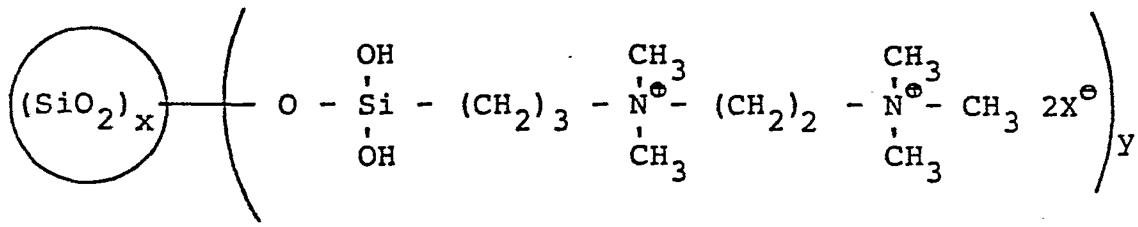

(X)

worin y 0,10 bis 0,5 mMol/g bedeutet und $X^{\ominus}$ die in Anspruch 2 gegebene Bedeutung besitzt.

5. Verbindungen nach Anspruch 1 der Formel XI

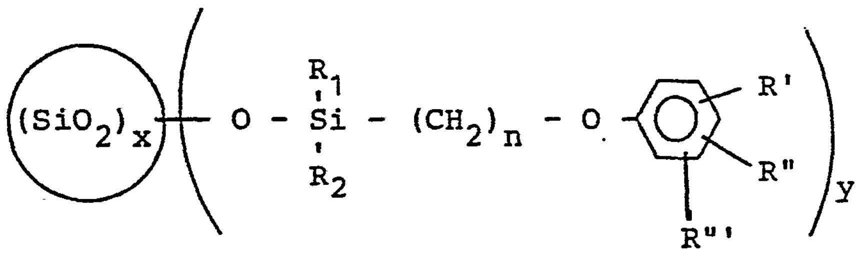

(XI)

worin n 1 bis 8, vorzugsweise 3, R', R" und R"' Wasserstoff oder Substituenten am aromatischen Ring, wie Halogene, $C_1$-$C_5$-Alkyl, $C_1$-$C_2$-Alkoxy, -CHO, -OH oder $-NH_2$-Gruppen sein können und y 0,1 bis 0,5 mMol/g bedeutet.

6. Verbindungen nach Anspruch 5 der Formel XII

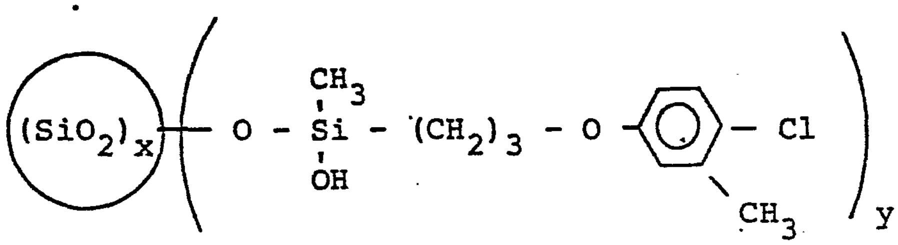

(XII)

worin y 0,1 bis 0,5 mMol/g bedeutet.

7. Verbindungen nach Anspruch 5 der Formel XIII

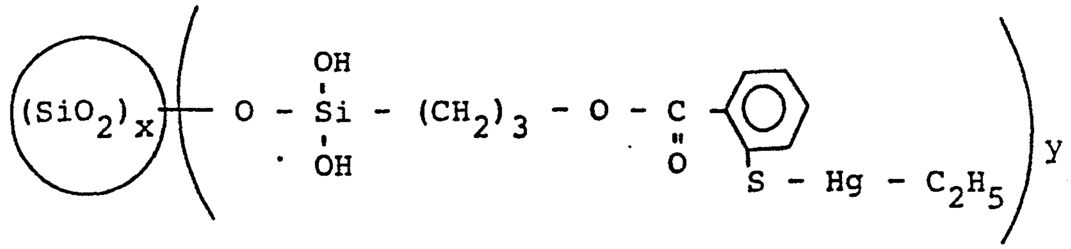

(XIII)

worin y 0,1 bis 0,5 mMol/g bedeutet.

8. Verbindungen nach Anspruch 1 der Formel XIV

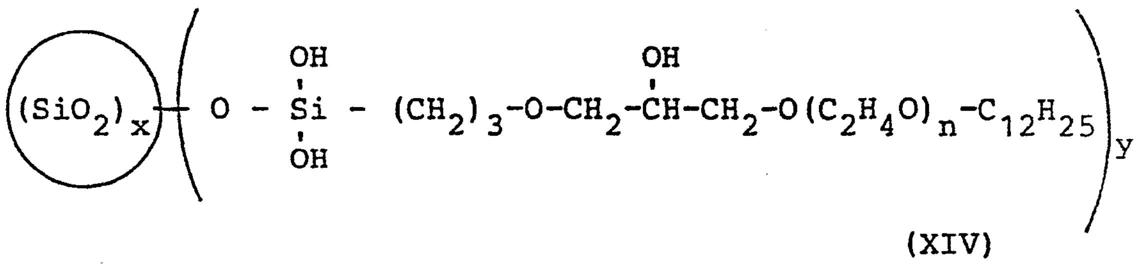

(XIV)

worin n 9 bis 12 und y 0,1 bis 0,4 mMol/g bedeuten.

9. Verbindungen nach Anspruch 1 der Formel XV

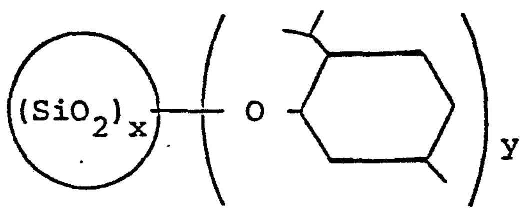

(XV)

worin y 0,1 bis 0,5 mMol/g bedeutet.

10. Verbindungen nach Anspruch 1 der Formel XVI

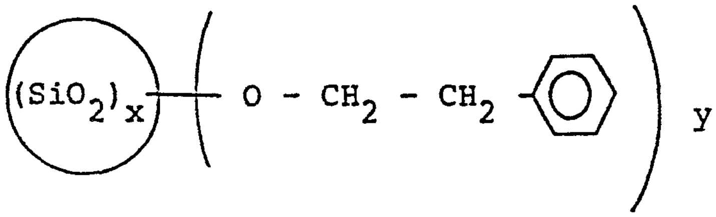

(XVI)

worin y 0,1 bis 0,5 bedeutet.

11. Verfahren zur Herstellung von an Siliciumdioxid als Träger gebundenen Wirkstoffen der folgenden Formeln I, II oder III

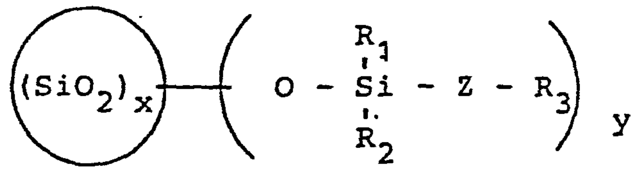

(I)

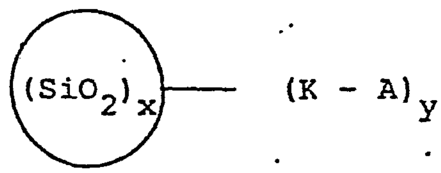

(II)

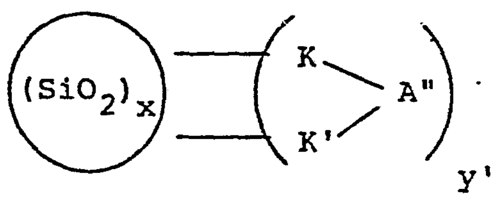

(III)

worin 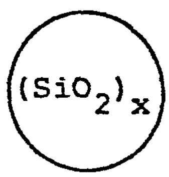 hochdisperse Teilchen bedeutet, y und y' die pro Menge oder Oberflächeneinheit des Trägers verankerten Gruppen in Mol bedeuten (mMol/g oder mMol/m²), y im Bereich von 0,1 bis 1 und y' im Bereich von 0,1 bis 0,5 liegen, Z $-(CH_2)_n-$, worin n für 0 bis 18 steht, oder einen Rest der Formeln

$$-[(CH_2)_3-O]_n, \quad -CH_2-\underset{OH}{CH}-CH_2-$$

oder

$$-HN-(CH_2)_3-NH-$$

bedeutet, worin n' für 1 bis 3 steht,

$R_1$ und $R_2$ gleich oder verschieden sein können und eine Methyl- oder eine Hydroxylgruppe bedeuten,

$R_3$ den einwertigen Rest eines Wirkstoffs bedeutet, mit der Maßgabe, daß, wenn Z eine Alkylengruppe bedeutet, in der n für 0 steht, der Wirkstoff unmittelbar mit einem seiner Kohlenstoffatome mit dem Siliciumatom verknüpft ist,

K und K' O, S oder >N-A' bedeuten, wobei in der Formel III die beiden Substituenten K und K' gleich oder unterschiedlich sein können,

A den einwertigen Rest eines Wirkstoffs der Formeln IV, V oder VI

H - S-A (IV)

H - O-A (V)

H - N-A (VI)
       |
       A'

bedeutet,

wobei A' ein Wasserstoffatom bedeutet oder wobei in der Formel VI A und A' zusammen mit dem Stickstoffatom, an das sie gebunden sind, den Rest eines Wirkstoffmoleküls bedeuten,

A" den zweiwertigen Rest eines Wirkstoffs der Formel VII

H - K \
          A" (VII)
H - K' /

bedeutet,

und wobei bei der Verankerung mit dem Träger bei den Wirkstoffen der Formel IV oder VI ein Wasserstoffatom durch

den Träger ersetzt wurde und bei dem Wirkstoff der Formel VII zwei Wasserstoffatome durch den Träger ersetzt wurden, dadurch g e k e n n z e i c h n e t , daß man

1) zur Herstellung von Verbindungen der Formel I

a) eine Verbindung der Formel XVII

$$R_4 - \underset{\underset{R_2}{|}}{\overset{\overset{R_1}{|}}{Si}} - Z - R_3 \qquad \text{(XVII)}$$

worin $R_1$, $R_2$ und $R_3$ die oben gegebenen Bedeutungen besitzen und $R_4$ eine $C_1$-$C_3$-Alkoxygruppe oder ein Chloratom bedeutet, mit der Oberfläche des Siliciumdioxids in an sich bekannter Weise kondensiert oder

b) einen reaktiven Spacer der folgenden Formel XVIII

$$R_4 - \underset{\underset{R_4}{|}}{\overset{\overset{R_4}{|}}{Si}} - Z - \text{funktionelle Gruppe} \qquad \text{(XVIII)}$$

in der $R_4$ die oben gegebene Bedeutung besitzt, an der Oberfläche der $SiO_2$-Teilchen durch Kondensation in an sich bekannter Weise verankert und danach den Wirkstoff am Spacer in einem oder mehreren Reaktionsschritten in an sich bekannter Weise einführt oder

c) eine Vorstufe des Wirkstoffs, gegebenenfalls über einen geeigneten Spacer, mit der Oberfläche des $SiO_2$ verknüpft und in einem weiteren Reaktionsschritt in die Wirkform gemäß folgender Gleichung umsetzt:

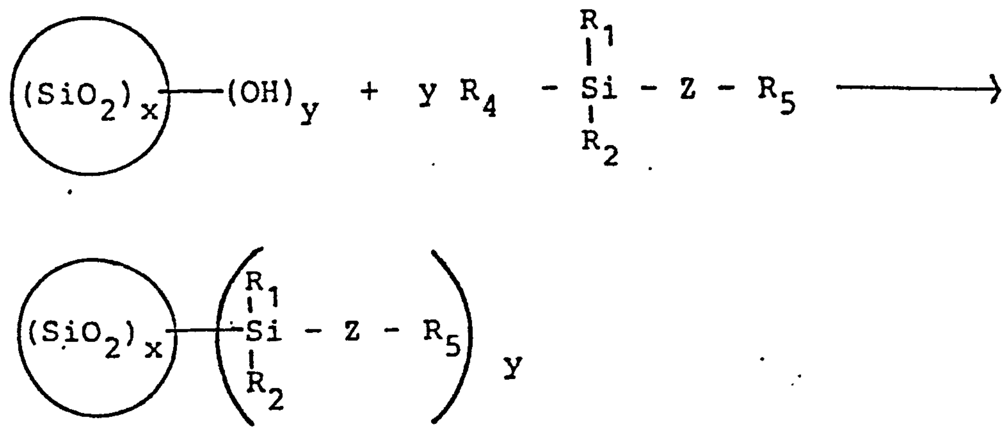

worin $R_1$, $R_2$ und $R_4$ die oben gegebene Bedeutung besitzen und $R_5$ die Vorstufe eines Wirkstoffrestes bedeutet

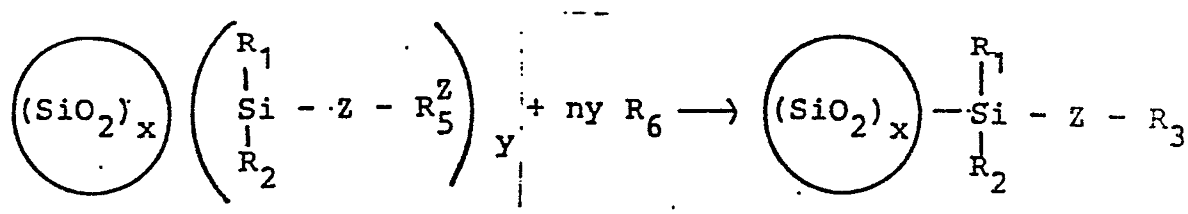

worin $R_6$ ein Reagenz zur Umwandlung der Wirkstoff-Vorstufe $R_5$ in die Wirkform $R_3$ bedeutet und $R_5$ mit η-Molekülen $R_6$, entsprechend der Wertigkeit von $R_5$ reagieren kann.

2) zur Herstellung von Verbindungen der Formel II wasserfreie Kieselsäure in an sich bekannter Weise in Benzol mit Thionylchlorid gemäß folgender Gleichung 1 umsetzt

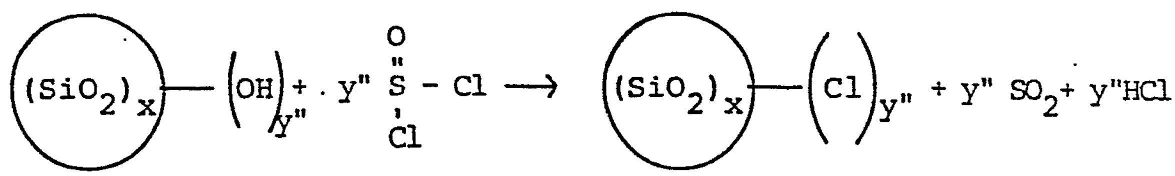

(1)

wobei y" die pro g oder m² an der $SiO_2$-Oberfläche umgesetzte Menge bedeutet, und nach Entfernung der flüchtigen Reaktionsprodukte $SO_2$ und HCl mit einer Verbindung der Formel H - K - A gemäß folgender Gleichung 2

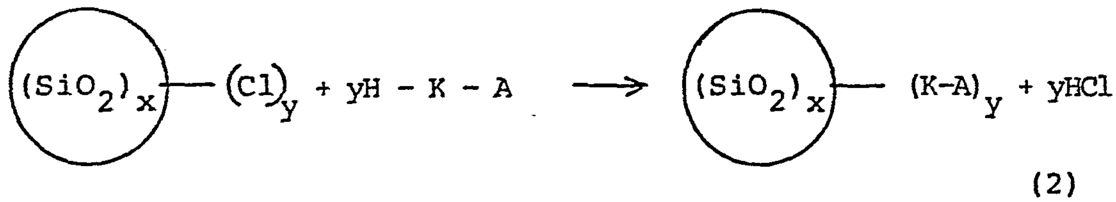

(2)

umsetzt, wobei $y \leq y''$, A und x die oben gegebenen Bedeutungen besitzen, und das Reaktionsprodukt in an sich bekannter Weise isoliert,

3) zur Herstellung von Verbindungen der Formel III Kieselsäure in an sich bekannter Weise in Benzol mit Thionylchlorid gemäß Gleichung 1 umsetzt und das erhaltene Reaktionsprodukt mit einer Verbindung der Formel gemäß folgender Gleichung 3

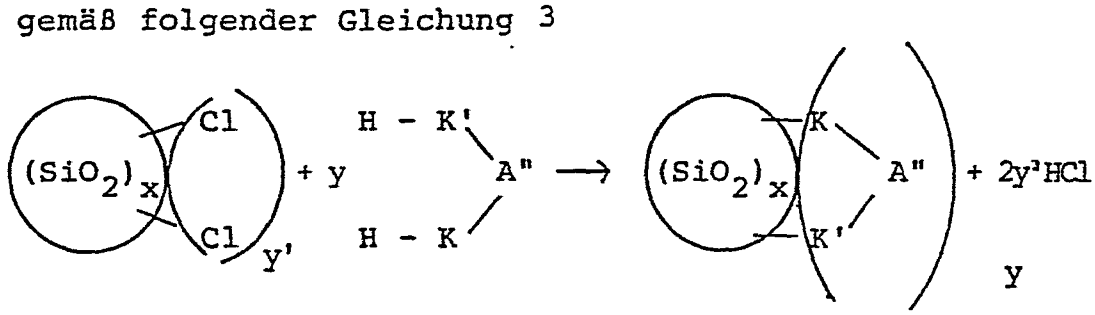

(3)

umsetzt, wobei $y' \leq \frac{y''}{2}$ bedeutet.

12. Verfahren nach Anspruch 11, dadurch gekennzeichnet, daß man bei der Herstellung von Verbindungen der Formel I den reaktiven Spacer oder den bereits mit dem Wirkstoff verbundenen reaktiven Spacer in der Weise an der Oberfläche des Trägers einführt, daß durch Einstellen eines physikalischen Adsorptionsgleichgewichts in einem ersten Reaktionsschritt in unpolarem Lösungsmittel (Temperatur 0 bis 20°C, vorzugsweise 15°C) ein definiertes Sorbat erzeugt wird und die chemische Verankerung nach Entfernung des Dispersionsmittels durch Temperaturerhöhung auf 50 bis 250°C, vorzugsweise 80 bis 200°C, erfolgt und durch nachfolgende Behandlung mit Wasser die am Spacer verbliebenen Ethoxy- bzw. Chlorsilanreste hydrolytisch zu SiOH-Gruppen umgewandelt werden.

13. Verfahren nach Anspruch 11, dadurch g e k e n n - z e i c h n e t , daß man bei der Herstellung von Verbindungen der Formeln II und III den Wirkstoff in der Weise an der Oberfläche des Trägers einführt, daß man am Träger in einem ersten Schritt chemisorbiert und danach mit dem Wirkstoff durch Einstellen eines physikalischen Adsorptionsgleichgewichts in einem zweiten Reaktionsschritt in unpolarem Lösungsmittel (Temperatur 0 bis 20°C, vorzugsweise 15°C) ein definiertes Sorbat erzeugt und danach die chemische Verankerung nach Entfernung des Dispersionsmittels durch Temperaturerhöhung auf 50 bis 250°C, vorzugsweise 80 bis 200°C, durchführt und das Reaktionsprodukt (Chemisorbat) im Vakuum und durch Waschen mit wasserfreien organischen Lösungsmitteln reinigt.

14. Verwendung der Verbindungen nach einem der Ansprüche 1 bis 10 als Desinfektionsmittel, geruchsbindende Substanz, spezifische Antidote für den peroralen Gebrauch, Lokalanästhetika, Lichtschutzmittel, als Arzneimittel mit retardierter Wirkstoffreisetzung, als Mittel, bei dem der unangenehme Geschmack oder Geruch des Wirkstoffs beseitigt oder abgemildert ist, und als stabilisierte Arzneiform von flüchtigen Arzneimitteln.

Europäisches Patentamt

# EUROPÄISCHER RECHERCHENBERICHT

0101027
Nummer der Anmeldung

EP 83 10 7729

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | FR-A-2 342 740 (PHARMACO) * Ansprüche 1-11; Seite 2, Zeilen 1-11; Seite 3, Zeilen 28-40; Seite 4, Zeilen 1-7; Seite 5, Zeilen 29-40; Seite 14, Zeilen 1-30 * | 1,11 | A 61 K 9/22<br>A 61 K 9/18 |
| X | EP-A-0 088 818 (BORDEN COMP.) * Ansprüche 1,2,7,8,10,12,13; Seite 39, Zeilen 9-29 * | 1,11 | |
| X | US-A-4 284 553 (R.J. BROWN) * Ansprüche 1,4,6,8 * | 1 | |
| Y | FR-A-2 364 686 (MERCK) * Ansprüche 1,4,6,8; Seite 1, Zeilen 1-5; Seite 4, Zeilen 31-38; Seite 5, Zeilen 1-4, 5-38; Seite 6, Zeilen 1-17; Seite 11, Zeilen 4-8, 12-24 * | 1 | RECHERCHIERTE SACHGEBIETE (Int. Cl. 3) |
| Y | FR-A-2 122 529 (RICHTER GEDEON) * Ansprüche 1,7-16,28; Seite 1, Zeilen 1-4; Seite 4, Zeilen 35-40 * | 1 | A 61 K<br>C 12 N |
| A | US-A-4 046 750 (CALIFORNIA INST. OF TECHNOLOGY) * Anspruch 1 * | 1 | |

--- -/-

Der vorliegende Recherchenbericht wurde für alle Patentansprüche erstellt.

| Recherchenort | Abschlußdatum der Recherche | Prüfer |
|---|---|---|
| DEN HAAG | 15-11-1983 | DEPIJPER R.D.C. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

# EUROPÄISCHER RECHERCHENBERICHT

0101027
Nummer der Anmeldung

EP 83 10 7729

Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | FR-A-2 407 937 (DYNAMIT NOBEL) * Anspruch 1 * | 1 | |
| | ----- | | |

RECHERCHIERTE SACHGEBIETE (Int. Cl. 3)

Der vorliegende Recherchenbericht wurde für alle Patentansprüche erstellt.

| Recherchenort | Abschlußdatum der Recherche | Prüfer |
|---|---|---|
| DEN HAAG | 15-11-1983 | DEPIJPER R.D.C. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82